# EUROPEAN PATENT APPLICATION

(11) **EP 3 159 003 A2**
(43) Date of publication of application: **26.04.2017**
(21) Application number: 16195360.9
(22) Date of filing: 24.10.2016
(51) Int. Cl.: A61K 36/746, A61K 36/185, A61K 36/19, A61K 36/28, A61K 36/53, A61K 36/60, A61P 19/02, A61P 19/10

(54) **COMPOSITION FOR ENHANCING BONE GROWTH, PREVENTING BONE RESORPTION DISORDERS AND FOR JOINT HEALTH**

(30) Priority: 23.10.2015 MY 1570381
(71) Applicant: Universiti Putra Malaysia, 43400 Serdang, Selangor (MY); Ministry of Agriculture, 62100 Putrajaya (MY)
(72) Inventor: MOHAMED, Suhaila, 43400 Serdang, Selangor (MY); NOR AIJRATUL ASIKIN MOHD, Shalan, 43400 Serdang, Selangor (MY); RUBIATUL, Adawiyah Bokhari, 43400 Serdang, Selangor (MY); TANTOWI, Nur Adeelah Che Ahmad, 43400 Serdang Selangor (MY); OSMAN, Wan Nurfarahin Wan, 43400 Serdang Selangor (MY)
(74) Representative: Carpmaels & Ransford LLP

(57) **Abstract**

The present invention discloses extracts Morinda, Orthosiphon, Clinicanthus, Ficus, Labisia and Gynurea spp. function to enhance bone growth, prevent bone resorption disorders and for joint health (anti-arthritis), and that when the Morinda, Orthosiphon, Clinicanthus, Ficus, Labisia and Gynurea extracts are administered to model rodent, osteopenia, arthritis, bone and joint ailments or bone fracture is treated or reduced, thereby completing the present invention.

## Description

### Field of the Invention

The present invention relates to a composition for enhancing bone growth, preventing bone resorption disorders and for joint health. More particularly, the present invention relates to a composition comprising Morinda, Orthosiphon, Clinicanthus, Ficus, Labisia and Gynurea spp. plant extracts for enhancing bone growth, preventing bone resorption disorders and for joint health.

### Background of the Invention

The strength and integrity of bones depends on maintaining a delicate balance between bone resorption by osteoclasts and bone formation by osteoblasts. As we age or as a result of disease, this delicate balancing act becomes tipped in favor of osteoclasts so that bone resorption exceeds bone formation, rendering bones brittle and prone to fracture. A better understanding of the biology of osteoclasts and osteoblasts is providing opportunities for developing therapeutics to treat bone ailments. Drugs that inhibit the formation or activity of osteoclasts are valuable for treating osteoporosis, Paget's disease, and inflammation of bone associated with rheumatoid arthritis or periodontal disease. Far less attention has been paid to promoting bone formation with, for example, growth factors or hormones, an approach that would be a valuable adjunct therapy for patients receiving inhibitors of bone resorption.

Osteoporosis is characterized by low bone mass and deterioration of bone structure that causes bone fragility and increases the risk of bone fracture. For practical purposes, the World Health Organization (WHO) has defined osteoporosis as a bone mineral density (BMD) value more than 2.5 standard deviations below the mean for normal young White woman. Individuals with osteoporosis are at high risk of suffering one or more fractures, injuries that can often be physically debilitating and potentially lead to a downward spiral in physical and mental health. Generalized osteoporosis is the most common form of the disease, affecting the whole skeleton. Osteoporosis can also occur in localized parts of the skeleton as a result of injury or conditions that reduce muscle forces on the bone, such as limb paralysis. There a variety of different types of osteoporosis. The most common form of osteoporosis is known as primary osteoporosis, which is not caused by some other specific disorder. Bone loss caused by specific diseases or medications is referred to as secondary osteoporosis.

The primary goal in treating people with osteoporosis is preventing fractures. A comprehensive treatment program includes a focus on proper nutrition, exercise, and prevention of falls that may result in fractures. The doctor may also prescribe one of several medications that have been shown to slow or stop bone loss or build new bone, increase bone density, and reduce fracture risk. If medication to prevent or treat osteoporosis is consumed, it is still essential that to obtain the recommended amounts of calcium and vitamin D. Exercising and maintaining other aspects of a healthy lifestyle are also important.

For people with osteoporosis resulting from another condition, the best approach is to identify and treat the underlying cause. If a medication that causes bone loss is consumed, the doctor may be able to reduce the dose of that medication or switch to another medication that is effective but not harmful to the bones. If a patient has a disease that requires long-term glucocorticoid therapy, such as rheumatoid arthritis or lupus, certain medications approved for the prevention or treatment of osteoporosis associated with aging or menopause can be consumed. Staying as active as possible, eating a healthy diet that includes adequate calcium and vitamins, and avoiding smoking and excess alcohol use are also important for people with osteoporosis resulting from other conditions. Children and adolescents with such conditions as juvenile rheumatic diseases and asthma can also be diagnosed with this kind of osteoporosis.

Some of the medicines listed below to treat osteoporosis and other bone related disorders have side effects.

*Bisphosphonates:* Several bisphosphonates are approved for the prevention or treatment of osteoporosis. These medications reduce the activity of cells that cause bone loss.

*Parathyroid hormone:* A form of human parathyroid hormone (PTH) is approved for postmenopausal women and men with osteoporosis who are at high risk for having a fracture. Use of the drug for more than 2 years is not recommended.

*RANK ligand (RANKL) inhibitor:* A RANK ligand (RANKL) inhibitor is approved for postmenopausal women with osteoporosis who are at high risk for fracture.

*Estrogen agonists*/*antagonists:* An estrogen agonist/ antagonist (also called a selective estrogen receptor modulator or SERM) is approved for the prevention and treatment of osteoporosis in postmenopausal women. SERMs are not estrogens, but they have estrogen-like effects on some tissues and estrogen-blocking effects on other tissues.

*Calcitonin:* Calcitonin is approved for the treatment of osteoporosis in women who are at least 5 years beyond menopause. Calcitonin is a hormone involved in calcium regulation and bone metabolism.

*Estrogen and hormone therapy:* Estrogen and combined estrogen and progestin (hormone therapy) are approved for the prevention of postmenopausal osteoporosis as well as the treatment of moderate to severe hot flashes and vaginal dryness that may accompany menopause. Estrogen without an added progestin is recommended only for women who have had a hysterectomy (surgery to remove the uterus), because estrogen increases the risk of developing cancer of the uterine lining and progestin reduces that risk.

Alternative therapies include the use of isoflavones that are naturally occurring compounds found in soybeans. Because they are structurally similar to estrogen, researchers have thought that they may hold promise as an alternative to estrogen therapy to protect postmenopausal women from osteoporosis. Several studies have explored the effects of soy isoflavones on bone health, but results have been mixed, ranging from a modest impact to no effect. Most of these studies had various limitations, including their short duration and small sample size, making it difficult to fully evaluate the impact of these compounds on bone health. Moreover, reports from NIH-supported clinical trials have failed to demonstrate a bone-sparing effect of soy isoflavones.

The use of plant extracts in traditional medicine can be traced back to 6000 years ago. In recent years, there has been a burgeoning use of plant extracts in traditional medicine, including Chinese Traditional Medicine (TCM) for modern drug discovery. These plant extracts are used for a variety of purposes including health improvement, beauty, and nutritional supplementation, as well as prevention and treatment of diseases, including bone related disease, osteoporosis, and arthritis.

*Clinacanthus nutans* is one of the candidate plants, which belongs to the Acanthaceae family. It is commonly known as Belalai gajah (Malay), and Phaya yo (Thai) and has been reported to have medicinal properties towards various diseases or disorders including (i) inflammatory conditions, for example haematoma, contusion, strain and sprains of injuries and rheumatism; (ii) dysentery; (iii) diabetes; (iv) fever; (v) anaemia; (vi) relieving pain; and (vii) jaundice.

Extracts from *Clinacanthus nutans* also claimed to have anti-cancer properties, as disclosed in Chinese Patent Publication No. CN103877133.

Morinda leaf, Orthosiphon, Clinicanthus, Ficus, Labisia and Gynurea spp. is believed to have broad therapeutic effects including phytoestrogenic and health promoting activity.

For example, Claude Jarakae Jensen et.al. in European patent application number EP 1605901 A1 discloses selective inhibition of estrogen production and providing estrogenic effects in the human body. Specifically, it features a method of implementation directed to selectively inhibit estrogen production and providing estrogenic effects in a human body. A dietary supplement includes a processed *Morinda* product that is used to inhibit aromatase or aromatase enzymes that function to convert androgens to estrogens, inhibit receptors from binding with estrogen, and reduce and/or regulate estrogen production, as well as reduce the amount of estrogen produced within the body and regulating such production. The dietary supplement further provides estrogenic effects. This patent application discloses methods and compositions effectively function to treat estrogen-dependent cancers, and particularly inhibit, destroy, and reverse the effects of estrogen dependent cancerous tumors through the introduction into the body (e.g. ingesting) a safe, pre-determined dosage of a nutraceutical composition formulated with or comprising one or more processed *Morinda* products for a safe, predetermined duration.

WO2007076024 discloses *Morinda* fruit based compositions for treatment of anti-inflammatory diseases through inhibition of COX-1, COX-2, INTERLEUKIN-1B, INTERLEUKIN-6, TNF-A, HLE, AND INOS. It was another object of some embodiments of that invention to provide a nutraceutical formulation comprising one or more processed *Morinda* products that function as the active ingredient in the nutraceutical formulation. The *Morinda* based composition is used for treatment of anti-inflammatory diseases through inhibition of COX-1, COX-2, INTERLEUKIN-1B, INTERLEUKIN-6, TNF-A, HLE, AND INOS.

KR20039720050214 discloses a therapeutic agent for osteoporosis, comprising *Ginkgo biloba* leaf extract as an active ingredient. The Ginkgo biloba leaf extract contains a large amount of phytoestrogen such as quercetin and kaempferol and thus can be usefully utilized as a therapeutic agent for treatment of osteopenia without having adverse effects of causing breast cancer.

Although the prior art makes mention of the use of *Morinda leaf, Orthosiphon, Clinicanthus, Ficus, Labisia and Gynurea spp.* for various therapeutic effects, there is no mention of their use in augmenting healthy bone growth, preventing bone resorption and promoting joint health (anti-arthritis).

The realization that medicinal plants provide a cheaper means of disease management, particularly bones related, has generated interest in their use globally, and has driven the search for newer therapies as done in the current study. Many of these plants have been used solely based on a traditional hunch, and studies are now providing evidence of their efficacy. Moreover, concerns of the side effects and other adverse effects resulting from synthetic compounds are also factors why these medicinal plants are gaining attention. Hence, it is advantageous to provide plant extracts of Morinda, Orthosiphon, Clinicanthus, Ficus, Labisia and Gynurea for enhancing bone growth, prevent bone resorption disorders and for joint health, which can further be developed into nutraceuticals for management of bone related diseases, including osteoporosis and arthritis.

### Summary of the Invention

Accordingly, the present inventors have made extensive efforts to find a natural herbal substance for treating bone related disorders, which has less adverse effects. As a result, the present inventors have found that extracts Morinda, Orthosiphon, Clinicanthus, Ficus, Labisia and Gynurea spp. function to enhance bone growth, prevent bone resorption disorders and for joint health (anti-arthritis), and that when the Morinda, Orthosiphon, Clinicanthus, Ficus, Labisia and Gynurea extracts are administered to rodent bone and joint ailment models, the osteopenia, arthritis, bone and joint ailments are treated or reduced, thereby completing the present invention.

Thus, it is an object of the present invention to provide extracts of Morinda, Orthosiphon, Clinicanthus, Ficus, Labisia and Gynurea, which are natural herbal substance for treating or preventing osteopenia, arthritis, bone and joint ailments or bone fracture, including enhancing bone growth, preventing bone resorption disorders and promoting joint health (anti-arthritis),

It is another object of the present invention to provide a pharmaceutical composition for treating or preventing osteopenia, arthritis, bone and joint ailments or bone fracture, including enhancing bone growth, preventing bone resorption disorders and joint health (anti-arthritis), which contains the Morinda, Orthosiphon, Clinicanthus, Ficus, Labisia and Gynurea spp. extracts.

It is also another object of the present invention to provide a method for preparing the Morinda, Orthosiphon, Clinicanthus, Ficus, Labisia and Gynurea *spp.* extracts.

In order to achieve the above objects, one aspect of the present invention provides a pharmaceutical composition for preventing or treating osteopenia, arthritis, bone and joint ailments or bone fracture, including enhancing bone growth, preventing bone resorption disorders and joint health (anti-arthritis), which contains Morinda, Orthosiphon, Clinicanthus, Ficus, Labisia and Gynurea *spp.* extracts.

A second aspect of the present invention provides a health functional food for preventing or treating osteopenia, arthritis, bone and joint ailments or bone fracture, including enhancing bone growth, preventing bone resorption disorders and joint health (anti-arthritis), which contains Morinda, Orthosiphon, Clinicanthus, Ficus, Labisia and Gynurea extracts.

A third aspect of the present invention provides a method for preparing a Morinda, Orthosiphon, Clinicanthus, Ficus, Labisia and Gynurea *extract,* the method comprising the steps of:
(i) finely cutting a whole plant of a Morinda, Orthosiphon, Clinicanthus, Ficus, Labisia and Gynurea;
(ii) extracting the cut plant with a solvent selected from the group consisting of water, an organic solvent, and mixtures thereof, thereby obtaining an extract; and
(iii) filtering the extract and concentrating the filtered extract under reduced pressure, thereby obtainng a Morinda, Orthosiphon, Clinicanthus, Ficus, Labisia and Gynurea extract.

The method of the present invention may further comprise the step of:
(iv) purifying the Morinda, Orthosiphon, Clinicanthus, Ficus, Labisia and Gynurea extract obtained in step (iii), concentrating the purified extract under reduced pressure.

The Morinda, Orthosiphon, Clinicanthus, Ficus, Labisia and Gynurea *extract* extract prepared by the above-mentioned method has the following effects:
- enhancing healthy bone growth,
- prevents bone resorption,
- promotes joint health (anti-arthritis), and
- suppress /reduce inflammation.

A fourth aspect of the present invention provides the use of the Morinda, Orthosiphon, Clinicanthus, Ficus, Labisia and Gynurea *extract* in the manufacture of a medicament for preventing or treating osteopenia, arthritis, bone and joint ailments or bone fracture, including enhancing bone growth, preventing bone resorption disorders and joint health (anti-arthritis).

Other features and embodiments of the present invention will be more apparent from the following detailed description.

### Brief Description of the Drawings

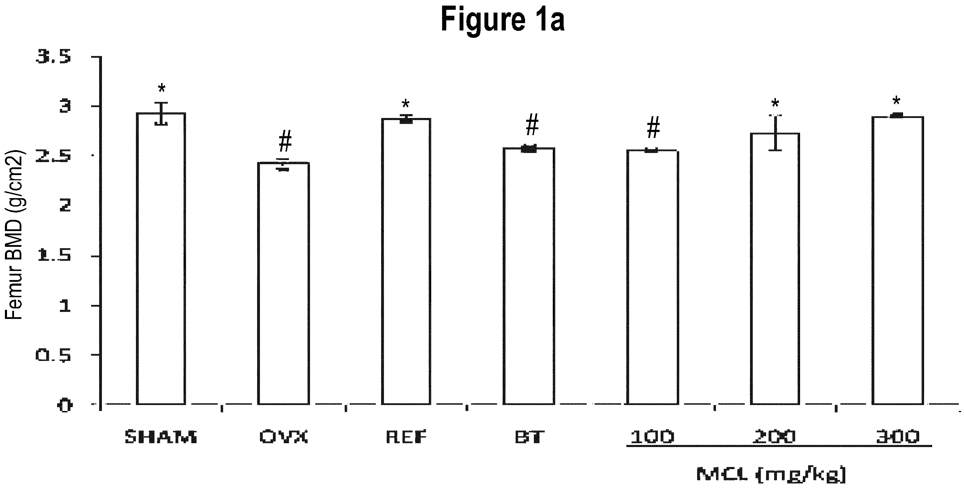

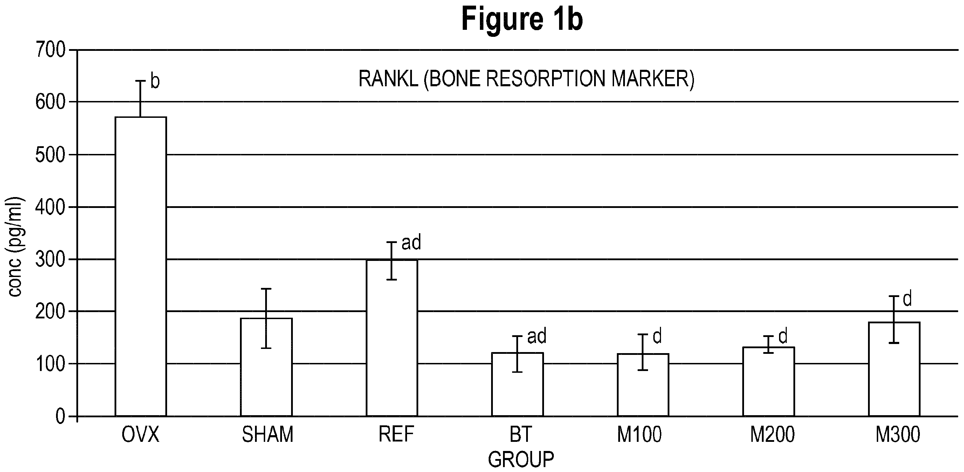

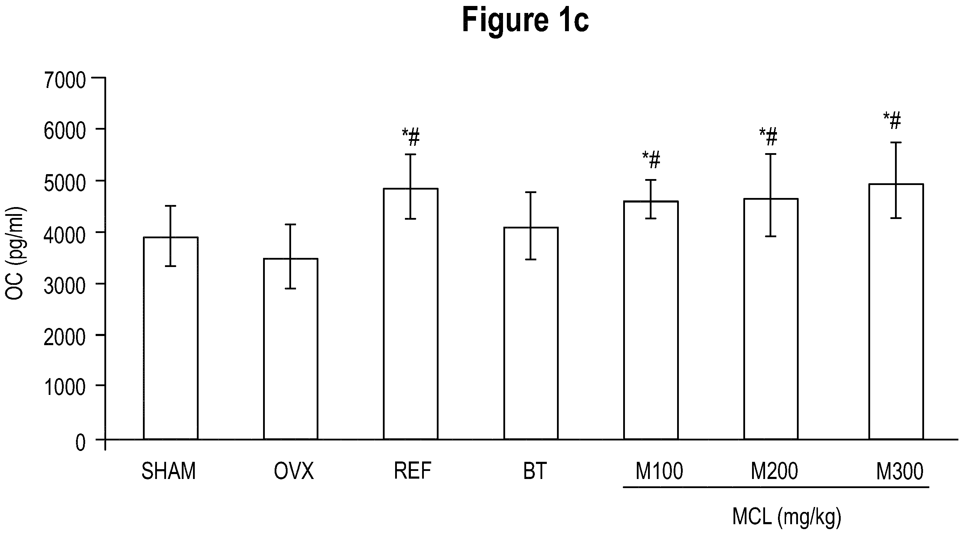

Figure 1 shows the effects of 16-weeks treatment with *Morinda* leaf extract (MCL), Remifemin (REF) or Black Tea (BT) on (a) bone mass density (BMD) in femur of ovariectomized (OVX) rats b micro-CT; (b) a bone resorption marker RANKL; and (c) Osteocalcin (OC) a bone formation marker. [Values are mean ± SD *P<0.05 vs. OVX; ^{#}P<0.05 vs. SHAM as evaluated by Duncan Test];

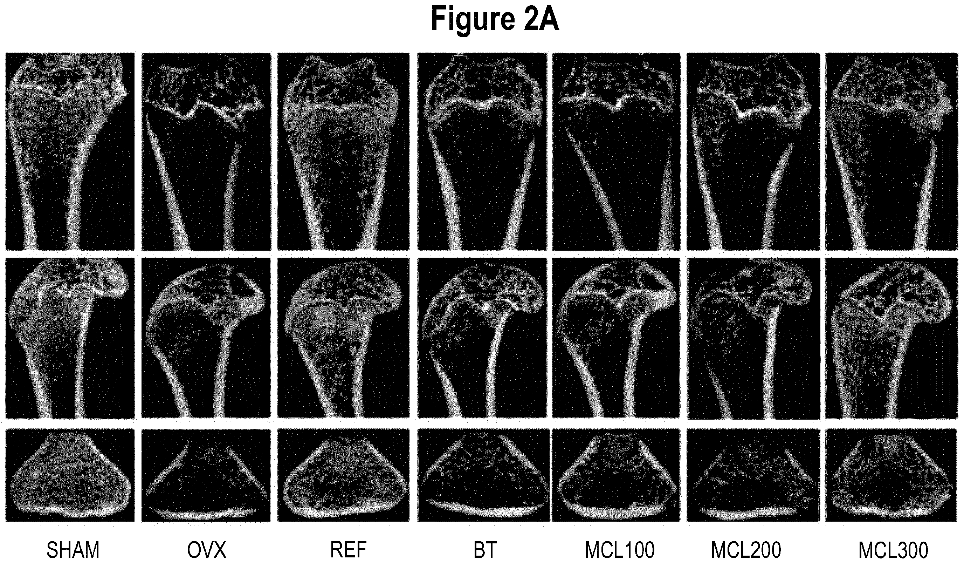

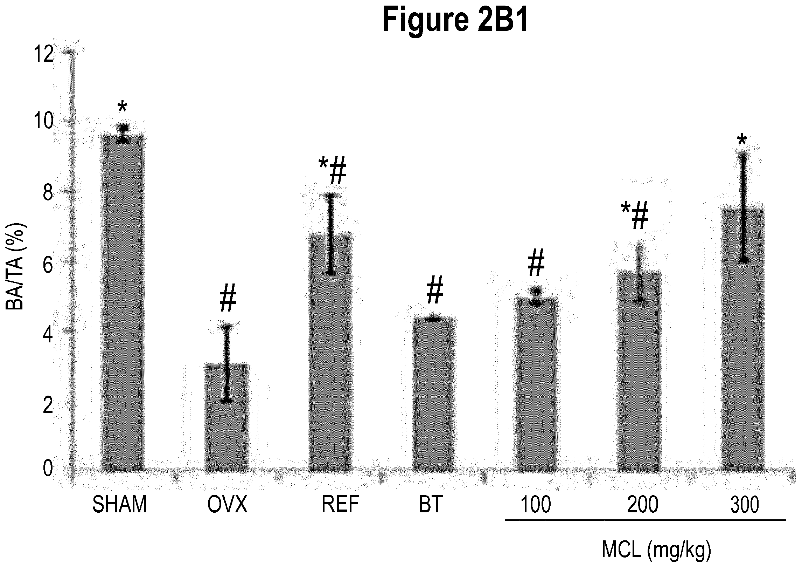

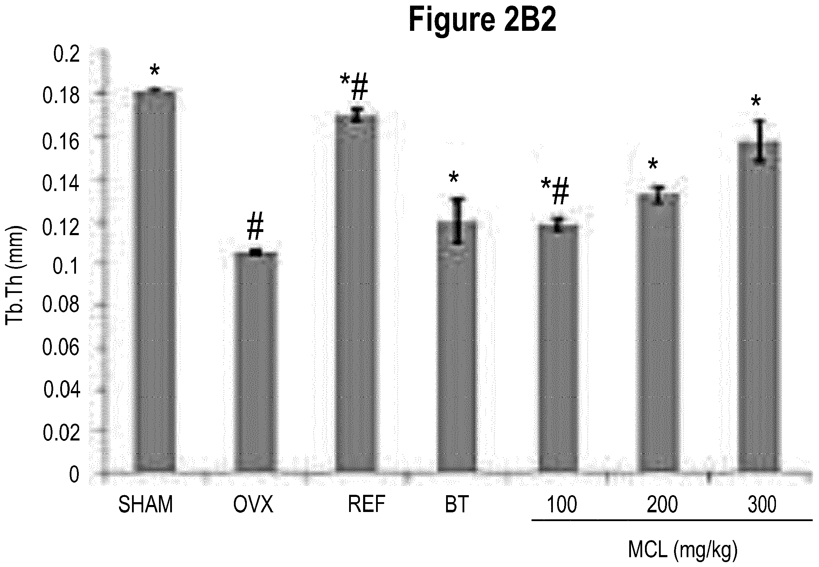

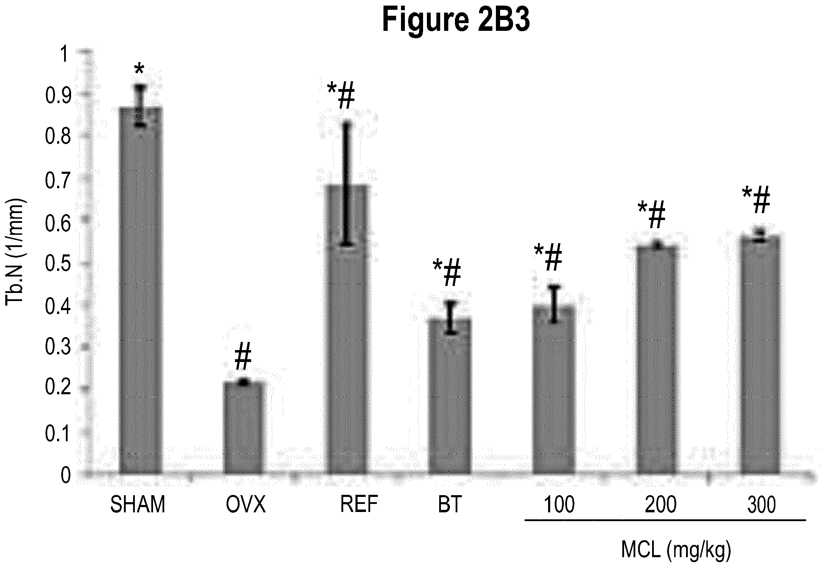

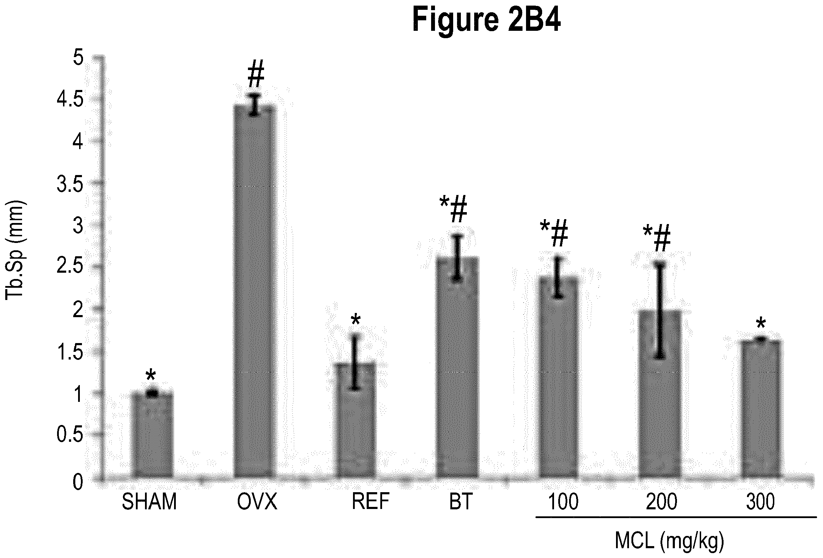

Figure 2 is a microCT analysis showing trabecula bone architecture (coronal, sagittal and transverse) within the metaphyseal femur region of ovariectomised mammals supplemented with Morinda leaf (MCL) extracts: (a) are the actual images; (b) digitalised analysis of BA/TA (bone area/tissue area), Tb.Th (Trabecular thickness), Tb.N (Trabecular number), and Tb.Sp (Trabecular separation) of ovariectomised mammals supplemented with Morinda leaf (MCL) extracts. (Values are mean ± SD *P<0.05 vs. OVX; ^{#}P<0.05 vs. SHAM as evaluated by Duncan Test);

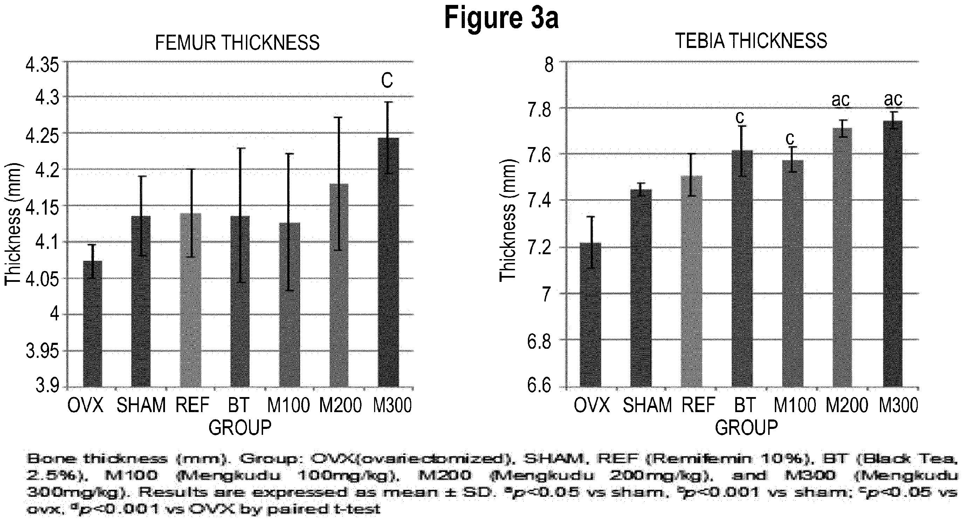

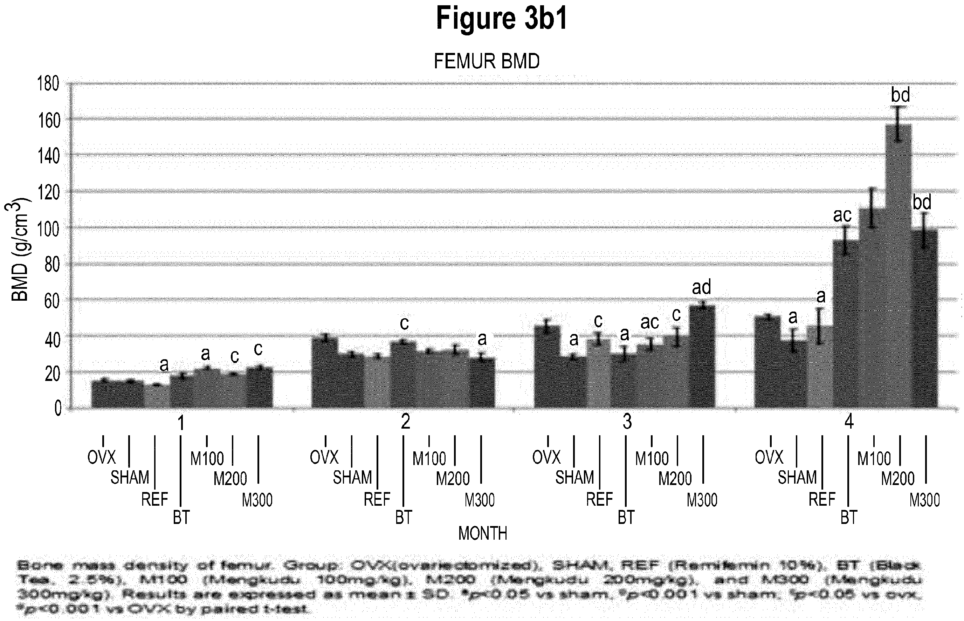

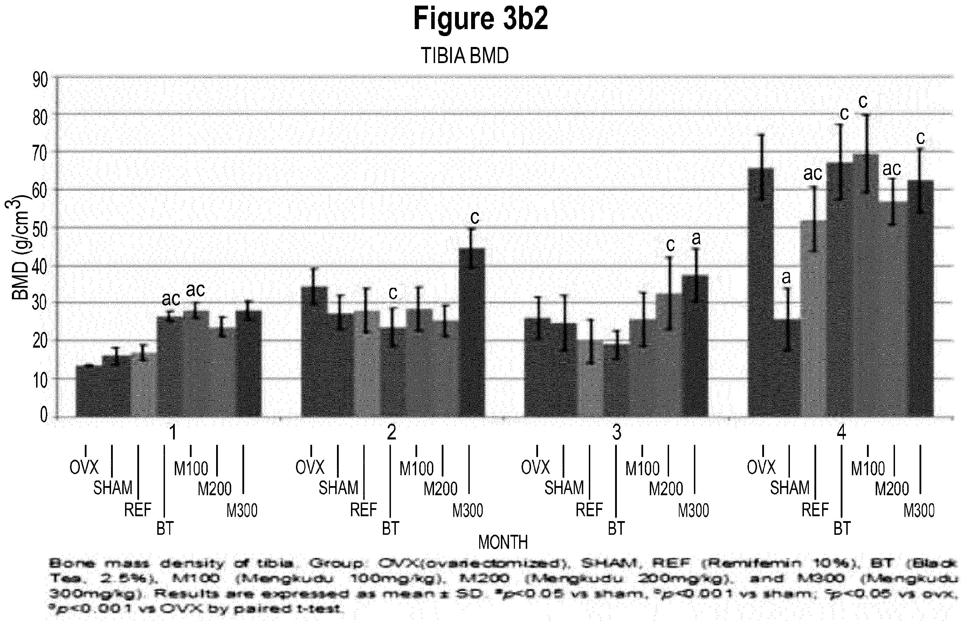

Figure 3 shows the bone growth effects of the herbs supplementation in ovariextomised rats (OVX) compared to control (Sham), according to the present invention on (a) femur and tibia thickness (b) Femur and tibia Bone mineral density;

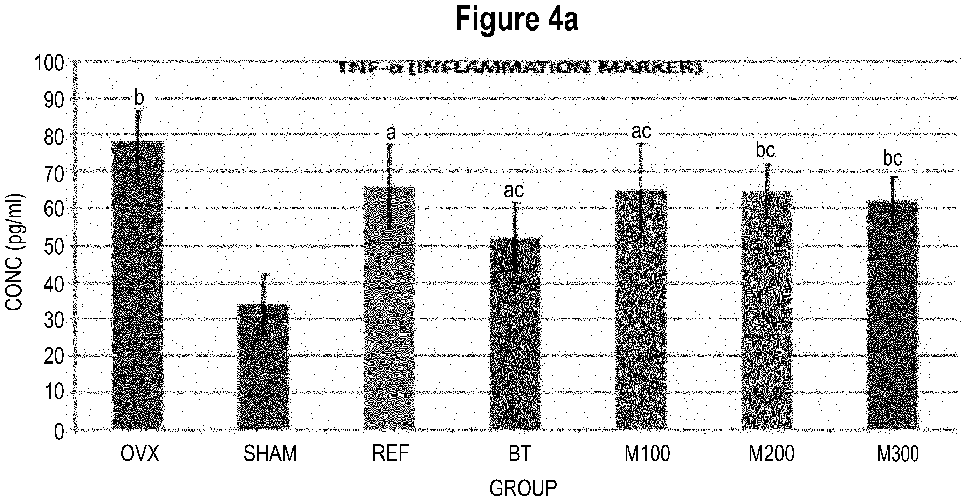

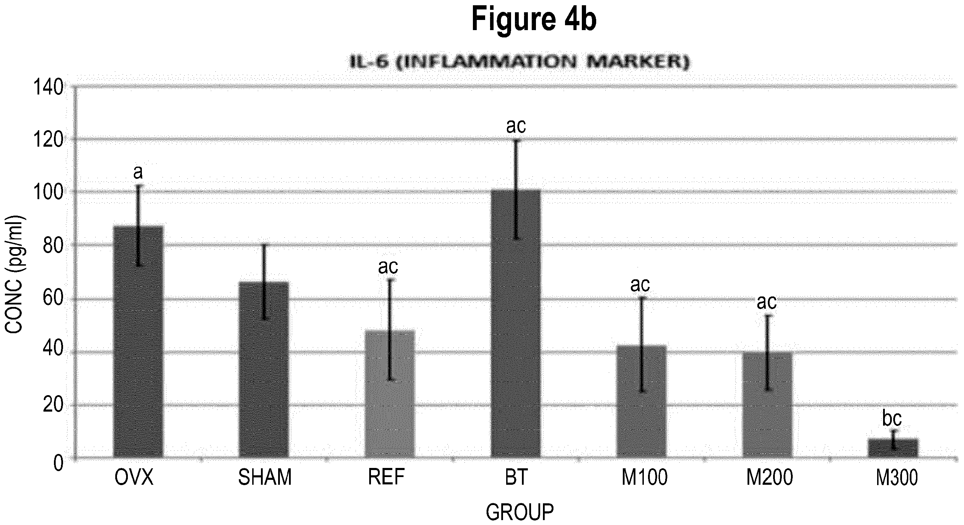

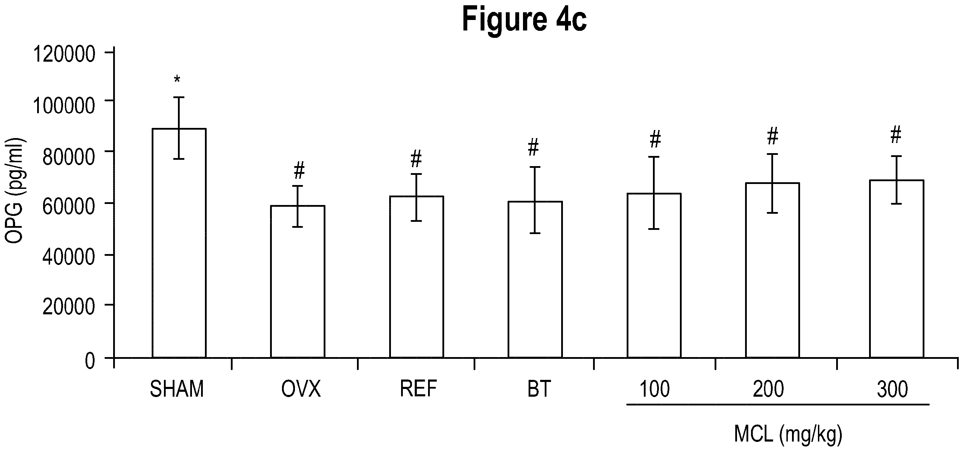

Figure 4 shows the effects of 16-weeks treatment of ovariectomized (OVX) rats given different treatments with *Morinda* leaf extract (MCL), Remifemin (REF) or Black Tea (BT) and compared with normal control (sham) on serum inflammation and biochemical markers i.e. (a) tumor necrosis factor alpha (TNF-α); (b) interleukin 6 (IL-6); and (c) Osteoprotegerin (OPG), also known as osteoclastogenesis inhibitory factor, (Value means are mean ± SD *P<0.05 vs. OVX; ^{#}P<0.05 vs. SHAM as evaluated by Duncan Test);

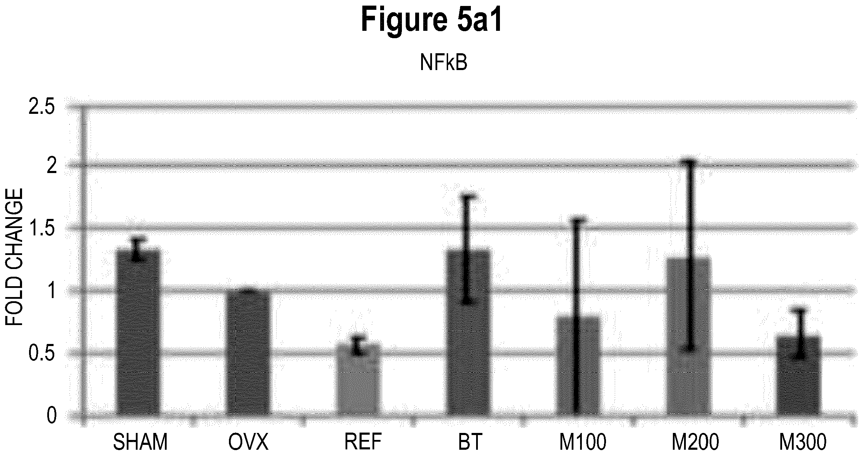

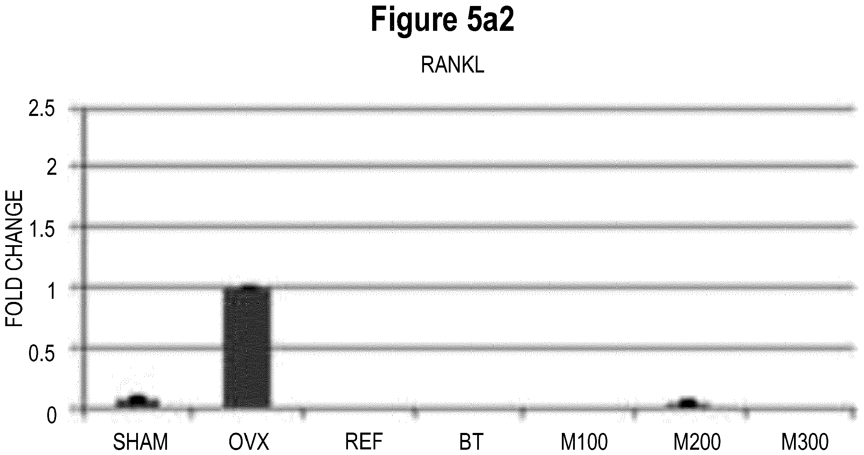

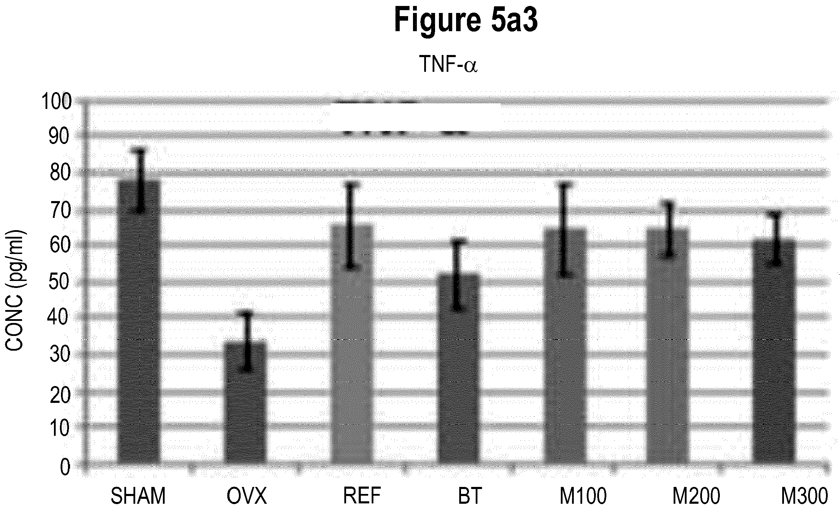

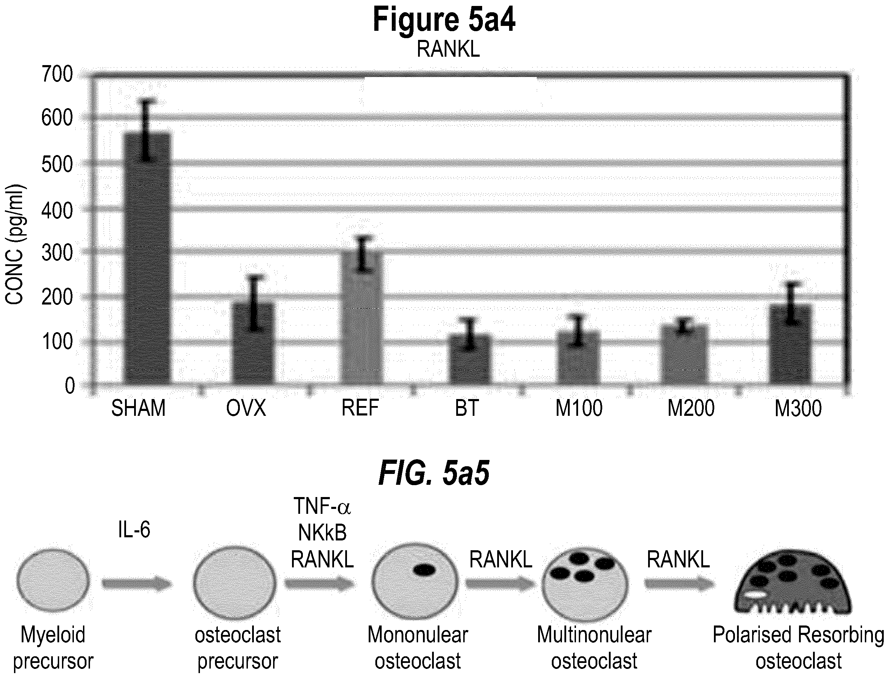

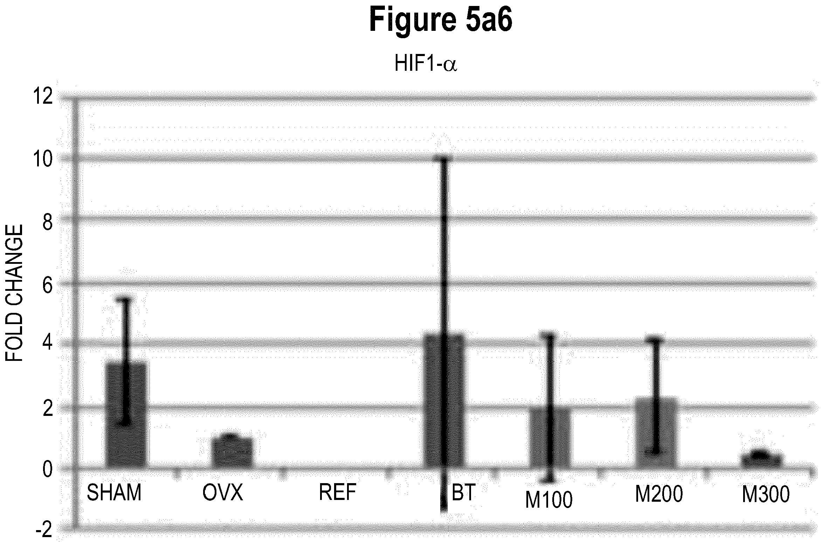

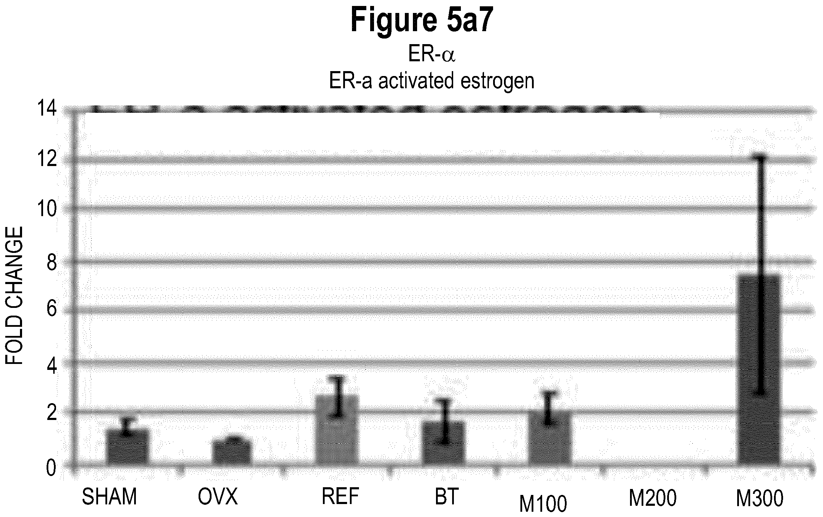

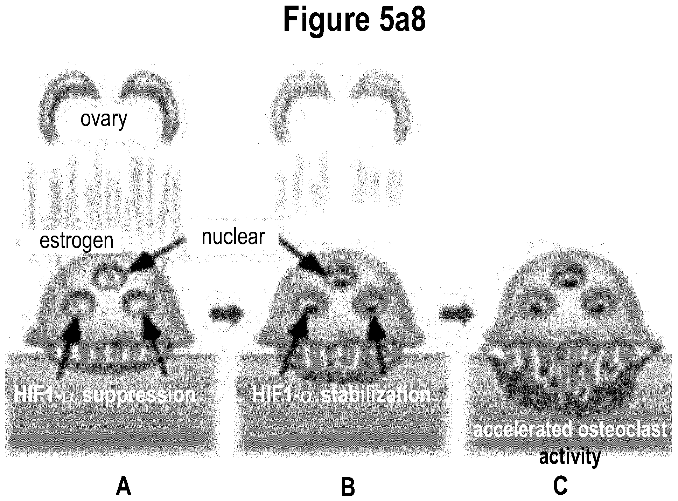

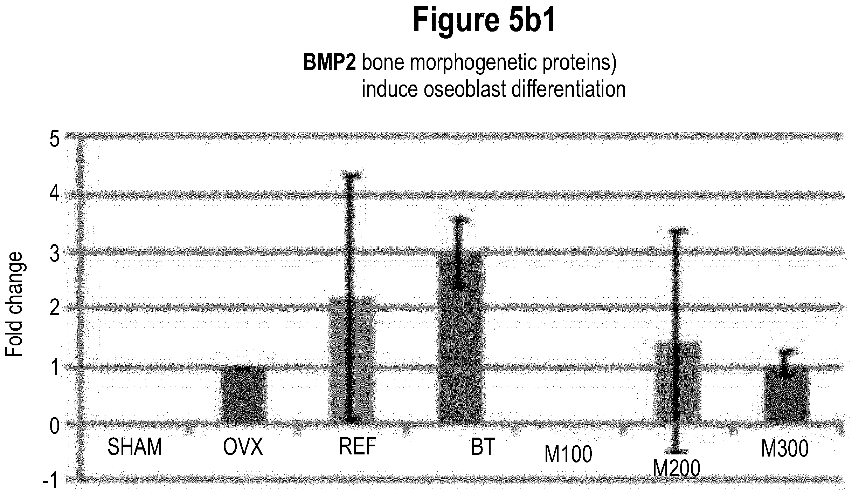

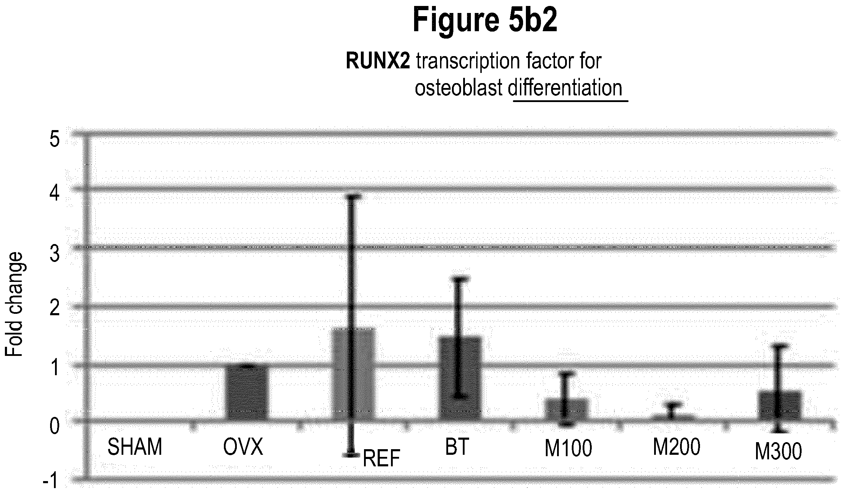

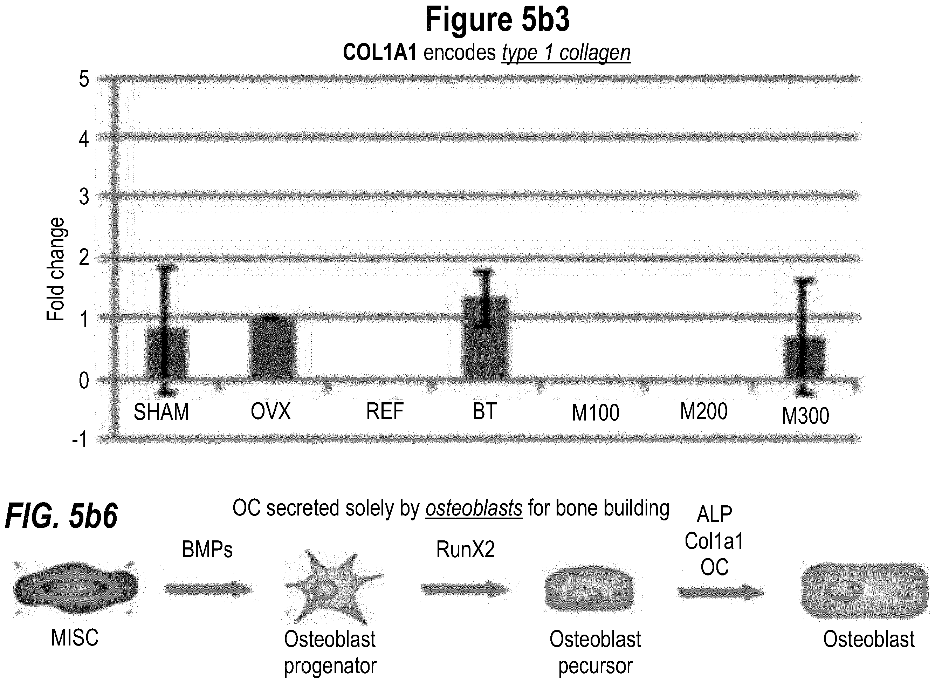

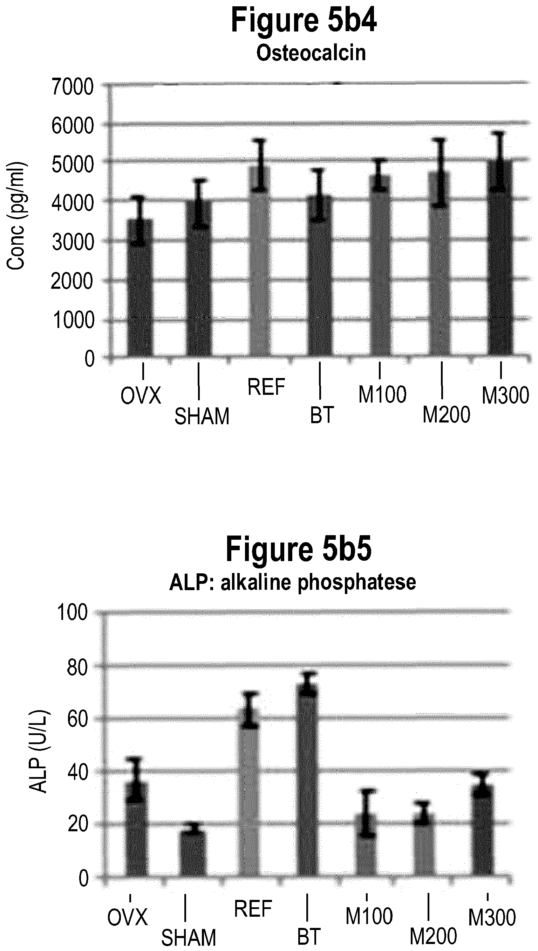

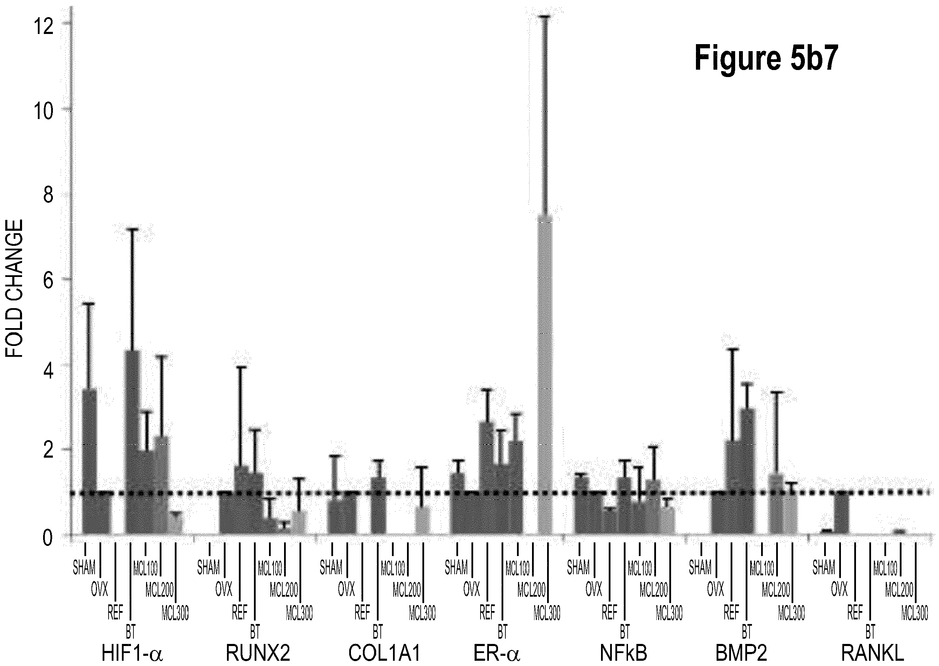

Figure 5 shows the effects of 16-weeks treatment with *Morinda* leaf extract (MCL), Remifemin (REF) or Black Tea (BT) on gene expression in tibiae of ovariectomized (OVX) rats: (a) nuclear factor kappa B (NFkB), Receptor Activator of Nuclear Factor Kappa B Ligand (RANKL), TNF-α, Hypoxia-inducible factor 1-alpha (HIF-1α), Estrogen receptor alpha (ER-α), and (b) Bone morphogenetic protein 2 (BMP-2), Runt-related transcription factor 2 (RUNX2), Colagen Type 1 Alpha 1 (COL1A1), Osteocalcin, ALP and finally the expression of all genes in each group normalized with the expressions in OVX+vehicle (OVX) group as represented by dash-line;

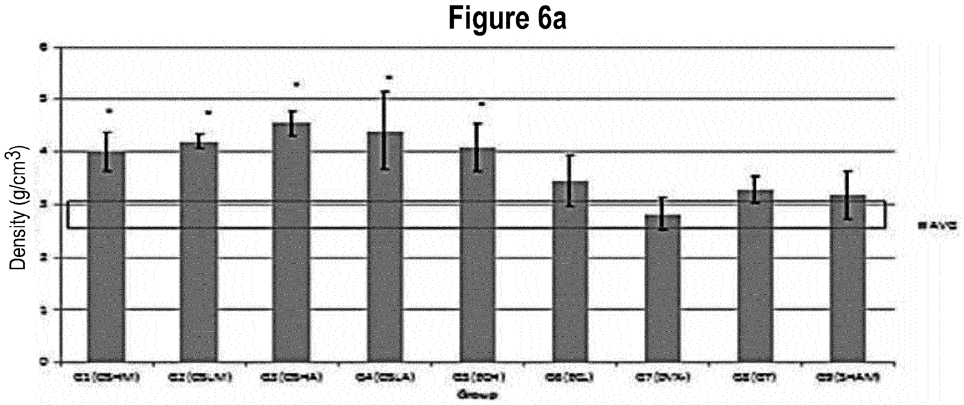

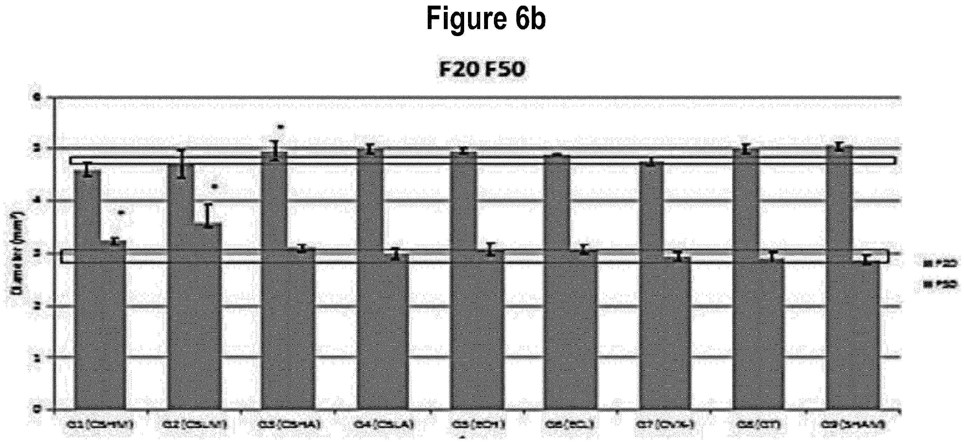

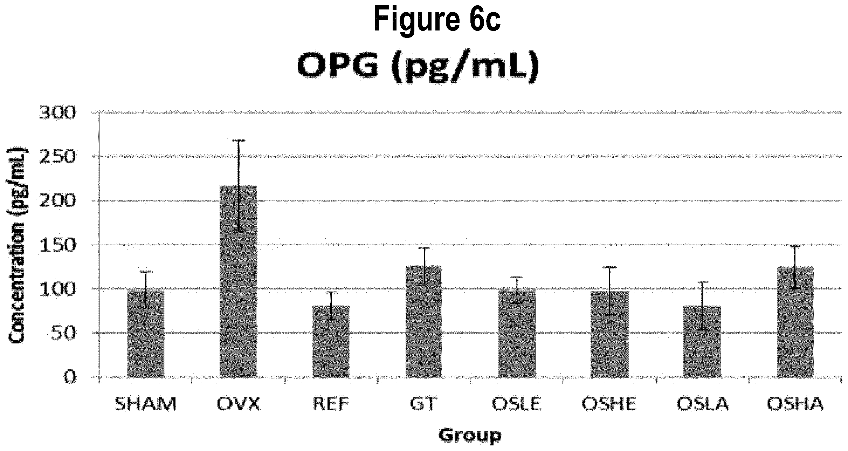

Figure 6 shows the bone growth effects of the *Orthosiphon spp.* supplementation in ovariextomised rats (OVX) compared to control (Sham), according to the present invention on (a) femur and tibia thickness (b) Femur and tibia Bone mineral density. [G1: SHAM (control group), G2 : OVX- ( negative control) , G3 : REF (Remifemin 100 mg/kg BW), G4 : GT (2% Green tea) , G5: OSLE (*Orthosiphon spp.* ethanol extract low dose 150 mg/kg BW), G6 : OSHE *(Orthosiphon spp.* ethanol extract high dose 300 mg/kg BW), G7 : OSLA (*Orthosiphon spp.* aqueous extract low dose 150 mg/kg BW), G8 : OSHA (*Orthosiphon spp.* aqueous extract high dose 300 mg/kg BW). OPG can reduce the production of osteoclasts by inhibiting the differentiation of osteoclast precursors]. They show the effects of 16-weeks treatment with *Orthosiphon spp. (OS)* leaf aqueous (OSA) or methanol (OSM) or ethanol (OSE) extract (L indicate low dose 150 mg/kg bw and H indicate high dose 300 mg/kg body weight), Remifemin (REF) or Green Tea (GT) on bone mass density and femur diameter of ovariectomized (OVX) rats;

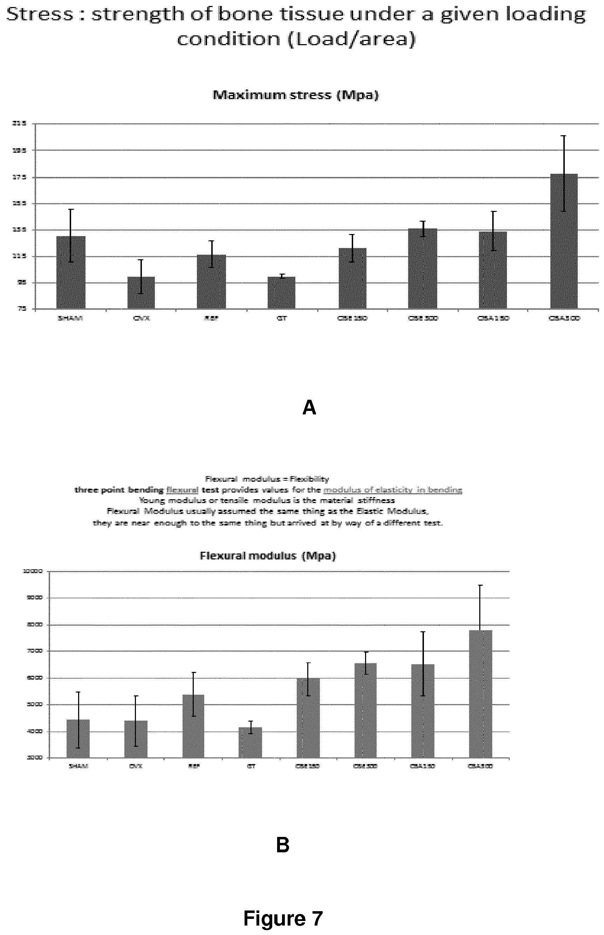

Figure 7 show the effects of the herbs (Orthosiphon spp.) after 4 month supplementation on bone strength (a) maximum stress (b) Flexural (flexibility) modulus.

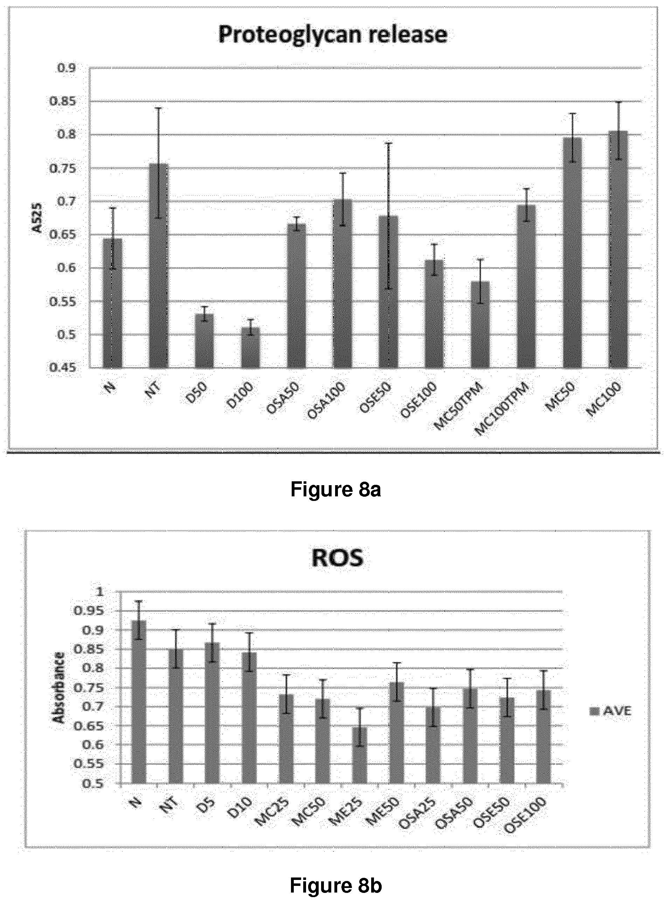

Figure 8 shows shows the effects of the herbs on (a) the Proteoglycan release and (b) the Reactive Oxygen Species (ROS) from bovine cartilage explant. N (Normal), NT (Non Treated), D50 (Drug, glucosamine low dose), D100 (Drug, glucosamine high dose), OSA 50 *(Orthosiphon spp.* aqueous extract low dose, 5 µl/well) OSA 100 (*Orthosiphon spp.* aqueous extract high dose, 10 µl/well), OSE 50 (*Orthosiphon spp.* 50% ethanol extract, 5 µl/well), OSE 100 *Orthosiphon spp.* 50% ethanol extract, 5 µl/well; MC (Morinda spp.);

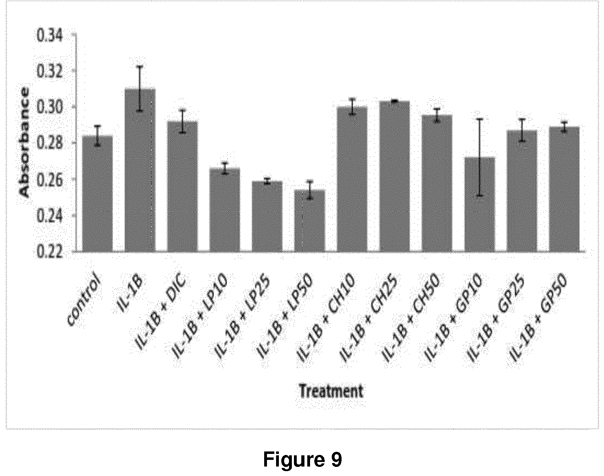

Figure 9 shows the effects of the other herbs on GAG release from cartilage explant in the presence of inflammatory cytokine IL-1B compared to diclofenac and normal control (LP Labisia spp. GP Gynurea spp.; Citrus spp.);

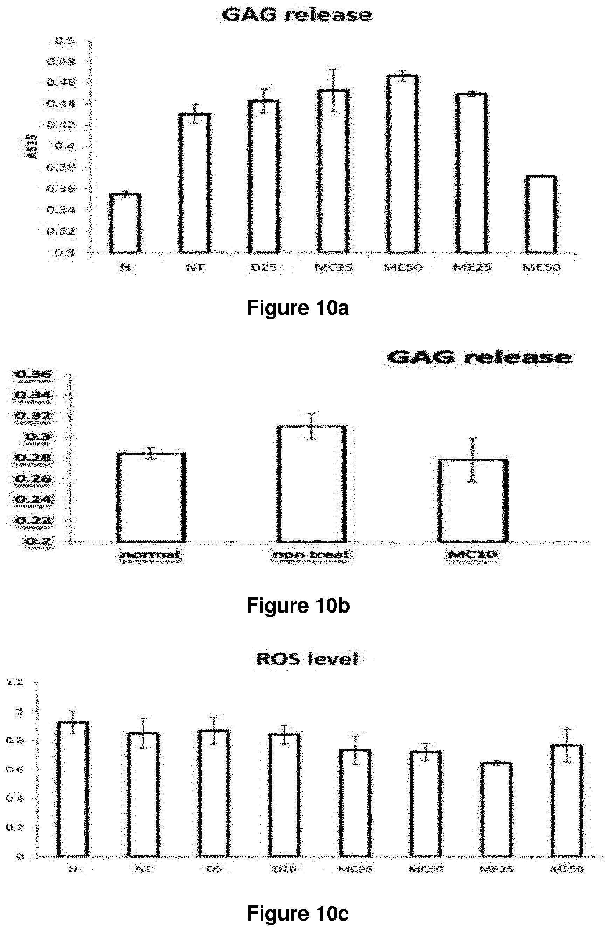

Figure 10 shows the effects of different Morinda species MC (citrifolia) and ME (elliptica) at different doses the inhibition of chondroitin degeneration markers from cartilage explants in the presence of IL-1B at diffent doses (ug/well); on (a) Glucoaminoglycan glycan (GAG) release (b) GAG and (c) ROS;

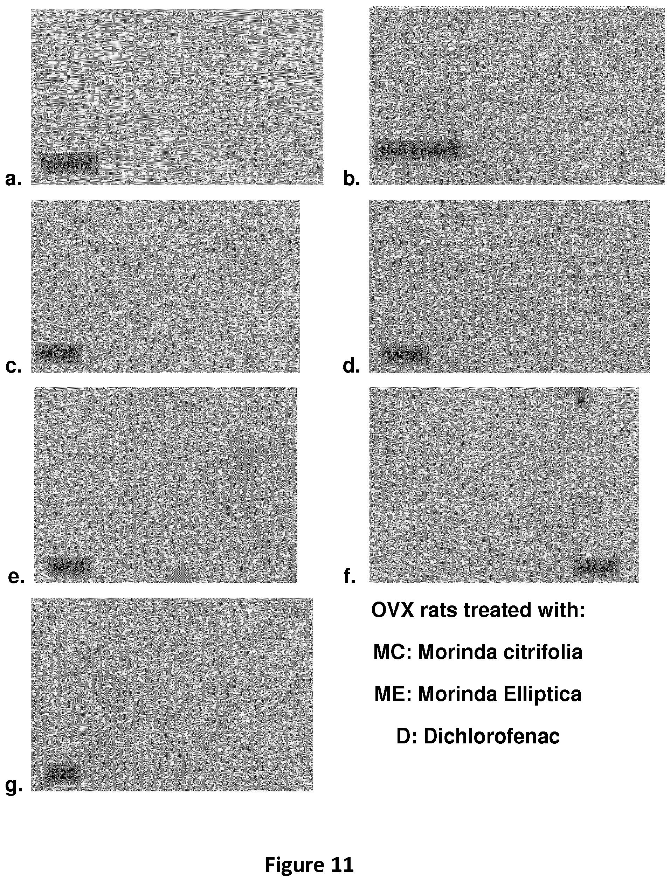

Figures 11 a to 11 g show the effects of the different Mroinda species at different doses compared to untreated OVX rats and normal control and OVX+dichlorofenac D25 observed histologically using H&E staining on cartilage explants;

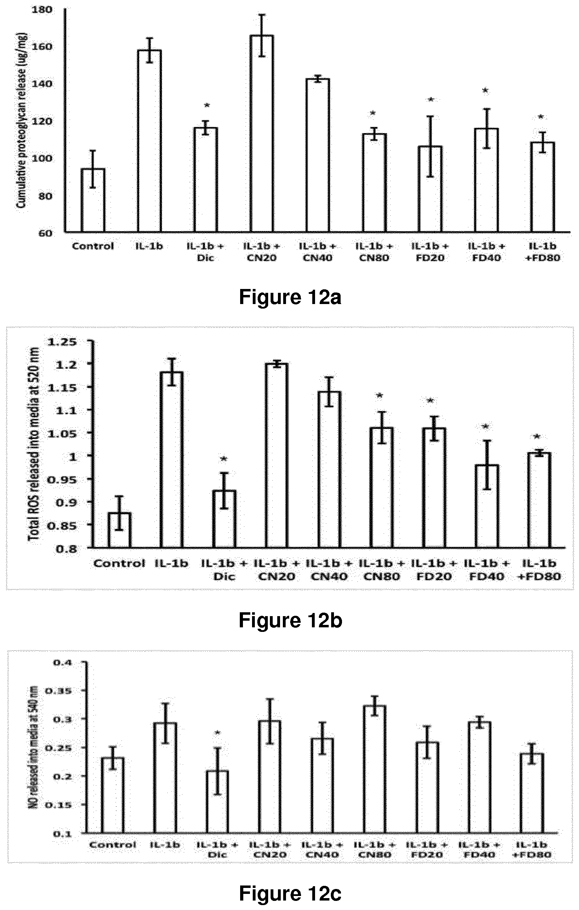

Figure 12a shows cumulative proteoglycan released in culture medium of cartilage explants induced with IL-1β (10 ng/ml) and supplemented with positive control Dichlorofenac (Dic), *Clinacanthus nutans* (CN) and *Ficus deltoidea* (FD) at 20, 40, and 80 µg/ml. The values were expressed as mean ± SEM, n=3. Sinificant differences to the IL-1β group was indicated by *P<0.05;
Figure 12b shows the total ROS released in culture medium of cartilage explants induced with 1 L-1 B (10ng/ml) and supplemented with positive control Dichlorofenac (Dic), *Clinacanthus nutans* (CN) and *Ficus deltoidea* (FD) at 20, 40 and 80 µg/ml. The values were expressed as mean ± SEM, n=3. Sinificant differences to the IL-1β group was indicated by *P<0.05;
Figure 12c shows the total NO released in culture medium of cartilage explants induced with 1 L-1 B (10ng/ml) and supplemented with positive control Dichlorofenac (Dic), *Clinacanthus nutans* (CN) and *Ficus deltoidea* (FD) at 20, 40 and 80 µg/ml. The values were expressed as mean ± SEM, n=3. Sinificant differences to the IL-1β group was indicated by *P<0.05; and

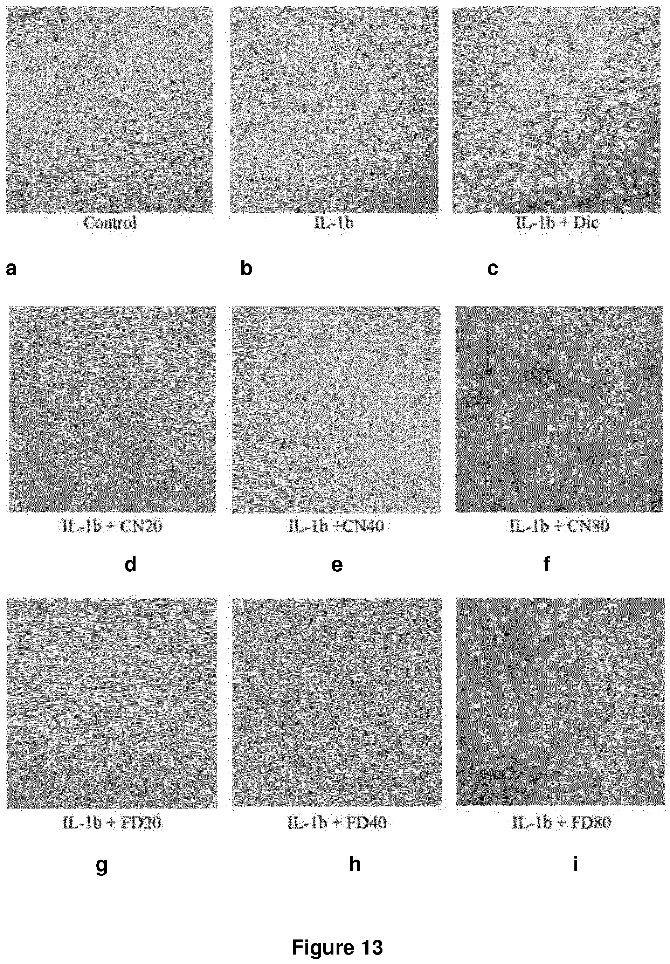

Figures 13a-13i are representive micrographs of toluidine blue stained explants (image taken at 1x magnification) to show cartilage protective effects by *Clinicanthus nutants* (CN); and *Ficus spp.*

### Detailed Description of the Invention

The present invention provides an extract of a plants from the Morinda leaf, Orthosiphon, Clinicanthus, Ficus, Labisia and Gynurea spp., having synergistic effects and activities of enhancing healthy bone growth, preventing bone resorption and promoting joint health (anti-arthritis) as well as suppressing inflammation in mammals, so that the extract can be effectively used for the prevention and treatment of osteopenia, arthritis, bone and joint ailments or bone fracture for use during health and bone growth retardation.

A person skilled in the art will readily appreciate that the present invention is well adapted to carry out the objects and obtain the ends and advantages mentioned, as well as those inherent therein. The embodiment describes herein is not intended as limitations on the scope of the invention.

The term "therapeutically effective amount" used herein throughout the specification refers to the amount of the active ingredient, the extract, to be administered orally to the subject to trigger the desired effect without or causing minimal toxic adverse effect against the subject.

One skilled in the art should know that the effective amount can vary from one individual to another due to the external factors such as age, sex, diseased state, races, body weight, formulation of the extract, availability of other active ingredients in the formulation and so on.

It is important to note that the extract used in the disclosure herein is derived from the plant species of Morinda leaf, Orthosiphon, Clinicanthus, Ficus, Labisia and Gynurea spp. The extracts obtained from the above-mentioned plant species are suitable to be incorporated into edible or topical products, or as capsules, ointments, lotions and tablets. The desired compounds to be extracted from the extract of the invention are mainly constituted of, but not limited to, biophenols, proteins, lipids, saccharides, minerals and small peptides. Due to polarity of these compounds, the polar solvent and water or alcohol is found to be effective in extracting these desired compounds from the plant matrix.

The extract or mixture of Morinda leaf, Orthosiphon, Clinicanthus, Ficus, Labisia and Gynurea spp. extracts may further include other sources of organic polyphenols and catechins, *scopoletin, sinensetin,* vitexin, isovitexin, rosmarinic acid, flavonoids and antioxidants derived from plants. Another embodiment of the present invention provides a pharmaceutical composition and comestible preparation that can be effectively used for the prevention and treatment of osteopenia, arthritis, bone growth disorders and bone fracture for use during health and bone growth retardation, the composition comprising solvent extracts derived from the parts of Morinda leaf, Orthosiphon, Clinicanthus, Ficus, Labisia and Gynurea spp. using an appropriate extraction solvent.

The pharmaceutical composition and comestible preparation may include a marine plant extract so that the composition containing mixture extract can be effectively used for the prevention and treatment of osteopenia, arthritis, bone resorption disorders and bone fracture for use during health and bone growth retardation. The composition containing mixture extract may further include other sources of organic polyphenols and catechins, *scopoletin, sinensetin,* flavonoids and antioxidants derived from a food sources.

The pharmaceutical preparation of the present invention can be formulated into therapeutic dosage forms and tablets, capsules, liquid orals, sterile injections, solvent or oily solutions or suspensions and the like. The preparation may be administered as food ingredients or as cosmeceuticals by known techniques, and oral and parenteral administration (including subcutaneous injection, intravenous or intramuscular technique), in dosage unit formulations containing conventional non-toxic pharmaceutically acceptable carriers, diluents or excipient. The extract of the parts of Morinda leaf, Orthosiphon, Clinicanthus, Ficus, Labisia and Gynurea spp., as it is in the preparation, may be a liquid, paste or a solid powder. As used herein, the term pharmaceutically acceptable carrier means a non-toxic, inert solid, semisolid or liquid filler, diluent, encapsulating material or formulation auxiliary of any type. Some examples of materials that can serve as pharmaceutically acceptable carriers are sugars and lactose, glucose, and sucrose; starches and corn starch and potato starch; cellulose and its derivatives and sodium carboxymethyl cellulose, ethyl cellulose and cellulose acetate; powdered tragacanth; malt; gelatin; talc; excipients and cocoa butter and suppository waxes; oils and peanut oil, cotton seed oil, safflower oil, sesame oil, olive oil, corn oil, and soybean oil; glycols, and propylene glycol; esters, and ethyl oleate and ethyl laurate; agar; buffering agents and magnesium hydroxide and aluminum hydroxide; alginic acid; pyrogen-free water; isotonic saline; Ringer's solution; ethyl water or alcohol; and phosphate buffer solutions, as well as other non-toxic compatible lubricants and sodium laryl sulfate and magnesium stearate, as well as coloring agents, releasing agents, coating agents, sweetening, flavoring and perfuming agents; preservatives and antioxidants can also be present in the composition, according to the judgment of the formulator.

The pharmaceutical and comestible preparation of the invention may be prepared by mixing the various components of the preparation using conventional methods. The preferred composition of the preparation may be prepared according to the constituent ranges set forth herein in Table 1. In an embodiment, the usual dose or therapeutically effective amount of the extract varies from about 0.1 to 5000 mg/kg of body weight of the patient per day. More preferably the usual dose or therapeutically effective amount of the extract is in the range of from about 5 to 500 mg/kg of body weight of the patient administered in equal portions twice a day or thrice a day.

The comestibles mentioned herein can be any common daily consumed processed food such as bread, noodles, confections, chocolates, beverages (for example instant tea preparation), and the like. One skilled in the art shall appreciate the fact that the aforesaid extract can be incorporated into the processed comestibles, capsules, tablets or topical medicine during the course of processing. Therefore, any modification thereon shall not depart from the scope of the present invention. As set forth in the above description, the pharmaceutical preparation with activities of enhancing healthy bone growth, prevents bone resorption and promotes joint health (anti-arthritis) as well as suppress inflammation in mammals, so that the preparation can be effectively used for the prevention and treatment of osteopenia, arthritis, bone growth disorders and bone fracture for use during health and bone growth retardation comprises water or solvent or alcoholic or solvent extract or their combinations from parts of Morinda leaf, Orthosiphon, Clinicanthus, Ficus, Labisia and Gynurea spp., or water or solvent or alcoholic or solvent mixture extract from Morinda leaf, Orthosiphon, Clinicanthus, Ficus, Labisia and Gynurea spp. and Lamiaceae *family.*

Preferably, the plant of Morinda leaf, Orthosiphon, Clinicanthus, Ficus, Labisia and Gynurea spp. is any one or combination of the plant species of *Morinda elliptica, Morinda umbellata, Morinda rigida, Orthosiphon spp., Labisia spp., Euricoma spp. Centella spp., Lamiaceae family spp., Ficus spp., Piper spp., Phillanthus spp., and Orthosiphon spp.*

The inventors of the present invention found that the water or alcohol or solvent extract derived from the aforementioned species possesses both acceptable taste that confers the derived extract to be comfortably incorporated with the comestibles product, capsules, tablets or topical medicine with minimal additional refining process. According to the preferred embodiment, the extract to be incorporated into the comestibles and medicine can be acquired from any known method not limited only to the foregoing disclosed method. Following another embodiment, the extract is prepared in a concentrated form, preferably paste or powdery form which enables the extract to be incorporated in various formulations of the comestibles, capsules, tablets or topical products. In line with the preferred embodiment, the extract shall be the plant metabolites which are susceptible to an extraction solvent. The compounds and small peptides with the bone growth enhancing properties, as well as suppress inflammation are those metabolites in the water or solvent or alcoholic extracts. Therefore, the water or solvent or alcoholic extracts of parts of Morinda leaf, Orthosiphon, Clinicanthus, Ficus, Labisia and Gynurea spp. or the water or solvent or alcoholic mixture extracts of parts of Morinda leaf, Orthosiphon, Clinicanthus, Ficus, Labisia and Gynurea spp. and *Lamiaceae family* is preferably derived from the extraction solvent of water, water or alcohol, acetone, chloroform, liquid CO₂ and any combination thereof. In view of the prominent property of encouraging bone growth by the extracts in a subject, further embodiment of the present invention includes a method comprising the step of administrating orally or topically to the subject an effective amount of an extract derived from the parts of Morinda leaf, Orthosiphon, Clinicanthus, Ficus, Labisia and Gynurea spp. or mixture extract derived from the parts of Morinda leaf, Orthosiphon, Clinicanthus, Ficus, Labisia and Gynurea spp. and *Lamiaceae family.*

The following examples are intended to further illustrate the invention, without any intent for the invention to be limited to the specific embodiments described therein.

### EXAMPLES

### Example 1

### Preparation of Morinda leaf, Orthosiphon, Clinicanthus, Ficus, Labisia and Gynurea spp. extract

*Plant parts* Morinda, Orthosiphon, Clinicanthus, Ficus, Labisia and Gynurea spp. were finely cut, washed, dried and extracted with water and/or organic solvents in the ratio range between (w/v) of 0:100 to 100:0. The solvents were then removed via any efficient technique, which include filtration and evaporation, in order to obtain a Morinda, Orthosiphon, Clinicanthus, Ficus, Labisia and Gynurea extract. The extract can be further purified, for example using activated carbon and concentrating the purified extract under reduced pressure.

### Example 2

### Preparation of composition containing mixture extract of Morinda leaf, Orthosiphon, Clinicanthus, Ficus, Labisia and Gynurea spp.

The extracts of Morinda, Orthosiphon, Clinicanthus, Ficus, Labisia and Gynurea spp. were tested individually or mixed in various ratios within various ranges and used in the present invention, as described herein.

### Example 3

### Test Analysis

### A) The effects of Morinda leaves (Morinda spp.) or Orthosiphon spp. extract on ovariectomy-induced osteoporosis.

*Material and methods*: Fifty six, 3-month-old female Sprague-Dawley rats were used and randomly assigned into sham-operated group (SHAM) and six ovareictomised (OVX) subgroups, i.e. OVX with vehicle (OVX); OVX with Remifemin (REF, 100mg/kg/day); OVX with black tea (BT, 2.5%/day); OVX with *Morinda* of graded doses (100, 200 or 300 mg/kg/day). Daily oral administration of *Morinda spp.,* 100mg/kg Remifemin or BT started on 4^{th} week after OVX for 16 weeks. Bone turnover, bone strength and molecular mechanism were analysed by biochemical markers, three-point bending test and real time PCR. The trabecular bone microarchitecture was evaluated by microcomputed tomography (μCT).

Figure 1 shows the effects of 16-weeks treatment with *Morinda* leaf extract (MCL), Remifemin (REF) or Black Tea (BT) on (a) bone mass density (BMD) in femur of ovariectomized (OVX) rats b micro-CT; (b) a bone resorption marker RANKL; and (c) Osteocalcin (OC) a bone formation marker. [Values are mean ± SD *P<0.05 vs. OVX; ^{#}P<0.05 vs. SHAM as evaluated by Duncan Test].

Bone physical parameters: Table 1 shows the femur and tibia bone weight in OVX group were significantly lower than the sham group. Supplementation with 100mg/kg Remifemin and *Morinda* (300mg/kg) increased the femur weight, and *Morinda* at 200mg/kg significantly increased the tibia weight than OVX group. The thickness of femur and tibia, and length of tibia also increased in OVX rats treated with *Morinda* (300mg/kg).

**Table 1 Effects of 16-weeks treatment with Morinda spp., 100mg/kg Remifemin or black tea on bone physical parameters of ovariectomized (OVX) rats.**

| Group | Bone weight (g) | | Bone Thickness (mm) | | Bone Length (mm) | |
|---|---|---|---|---|---|---|
| | Femur | Tibia | Femur | Tibia | Femur | Tibia |
| Sham | 1.04±0.03* | 0.99±0.02* | 4.14±0.06 | 7.45±0.03* | 34.89±0.94 | 40.18±0.64 |
| OVX | 0.91±0.04^{#} | 0.80±0.02^{#} | 4.07±0.02 | 7.22±0.11^{#} | 34.56±0.84 | 39.80±0.76 |
| Ref | 0.98±0.06* | 0.84±0.06^{#} | 4.14±0.06 | 7.51±0.09* | 34.29±1.17 | 40.47±1.06 |
| BT | 0.95±0.06^{#} | 0.84±0.06^{#} | 4.14±0.09 | 7.61±0.11*^{#} | 35.24±0.70 | 40.38±0.99 |
| MCL100 | 0.94±0.02^{#} | 0.83±0.04^{#} | 4.13±0.09 | 7.58±0.06* | 35.29±0.50 | 40.96±0.53 |
| MCL200 | 0.95±0.04^{#} | 0,88±0.03*^{#} | 4.18±0.09 | 7.71±0.04*^{#} | 35.52±1.13 | 41.46±0.47* |
| MCL300 | 0.98±0.04* | 0.85±0.04^{#} | 4.24±0.05* | 7.74±0.04*^{#} | 34.65±4.40 | 41.56±0.45* |

| | | | | | | |
|---|---|---|---|---|---|---|
| Values are mean ± SD *P<0.05 vs. OVX; ^{#}P<0.05 vs. SHAM as evaluated by Duncan Test. | | | | | | |

Wet weight, ash weight and Bone mineral content: There was no difference in the wet weight, dry weight and Bone mineral content (Ca, Zn and Mg) of tibia among any of the treatment groups, except black tea which had significantly higher wet and ash weight compared to OVX group, as shown in Table 2.

**Table 2 Effects of 16-weeks treatment with Morinda spp., 100mg/kg Remifemin or black tea on wet weight, ash weight and Bone mineral content in tibiae of ovariectomized (OVX) rats.**

| Group | Wet weight (g) | Ash weight (g) | Ca (mg/L) | BMC Zn (mg/L) | Mg (mg/L) |
|---|---|---|---|---|---|
| Sham | 0.63±0.02 | 0.24±0.02 | 36.25±0.11 | 1.87±0.09 | 4.59±0.005 |
| OVX | 0.54±0.01 | 0.21±0.004 | 36.50±0.51 | 2.11±0.27 | 4.60±0.01 |
| Ref | 0.58±0.04 | 0.23±0.02 | 37.27±0.57 | 1.90±0.07 | 4.60±0.02 |
| BT | 0.63±0.01* | 0.27±0.005* | 36.25±1.13 | 2.06±0.07 | 4.62±0.01 |
| MCL100 | 0.55±0.04 | 0.21±0.02 | 34.74±0-52 | 1.90±0.04 | 4.60±0.02 |
| MCL200 | 0.55±0.05 | 0.22±0.04 | 35.55±0.49 | 1.99±0.005 | 4.61±0.01 |
| MCL300 | 0.60±0.02 | 0.25±0.01 | 38.09±1.42 | 2.03±0.02 | 4.61±0.001 |

| | | | | | |
|---|---|---|---|---|---|
| Values are mean ± SD *P<0.05 vs. OVX; ^{#}P<0.05 vs. SHAM as evaluated by Duncan Test. | | | | | |

Bone mechanical properties: OVX resulted in a significantly decrease femur stiffness, as compared with SHAM group. Compared with the OVX group, treatment with *Morinda* at dose 300mg/kg significantly increased the femur stiffness (p<0.05). OVX also resulted in a decrease in maximum load and energy, and *Morinda* group (300mg/kg) was able to increase both parameters to the sham level, as shown in Table 3.

**Table 3 Effects of 16-weeks treatment with Morinda spp., 100mg/kg Remifemin or black tea on bone mechanical properties in the femoral diaphysis of ovariectomized (OVX) rats.**

| Group | Maximum Load (N) | Energy (N/mm) | Stiffness (N/mm) |
|---|---|---|---|
| Sham | 131.97±0.20 | 32.60±0.35 | 252.13±9.37* |
| OVX | 120.69±6.73 | 29.58±2.00 | 178.84±43.22^{#} |
| Ref | 126.84±10.71 | 31.45±1.00 | 215.10±2.47 |
| BT | 123.21±3.85 | 31.08±1.36 | 189.54±5.93^{#} |
| MCL100 | 125.87±9.52 | 31.43±2.21 | 187.10±23.91^{#} |
| MCL200 | 129.84±2.67 | 32.58±0.16 | 217.65±21.20 |
| MCL300 | 132.16±3.01 | 33.47±0.68* | 245.79±20.18* |

| | | | |
|---|---|---|---|
| Values are mean ± SD *P<0.05 vs. OVX; ^{#}P<0.05 vs. SHAM as evaluated by Duncan Test. | | | |

Figure 2 shows the bone growth effects of the extract of osteoporotic rats.

Micro-ct evaluation: A visualization of the femur metaphysis area revealed the massive loss of trabecular bone in OVX group. OVX-induced bone loss was clearly reduced in the femurs of *Morinda* (300 mg/kg) and 100mg/kg Remifemin group, as shown in Figure 2a. Morphometric analyses in OVX group revealed pronounced reductions in bone area per tissue area (BA/TA), trabecular thickness (Tb.Th), trabecular number (Tb.N) along with increased in trabecular separation (Tb.Sp). *Morinda* (200 and 300mg/kg) and 100mg/kg Remifemin group significantly increased BA/TA. All treatment groups were significantly increased the Tb.Th and Tb.N, and also significantly reduced the Tb.Sp, as compared to OVX group (see Figure 2b).

Figure 3 shows the bone growth effects of the herbs supplementation in ovariextomised rats (OVX) compared to control (Sham), according to the present invention on (a) femur and tibia thickness (b) Femur and tibia Bone mineral density.

Biochemical parameters of serum: All groups showed no increase in serum calcium and phosphate. However, *Morinda* at doses 100 and 200mg/kg significantly reduced serum ALP level, as shown in Table 4.

**Table 4 Effects of 16-weeks treatment with Morinda spp., 100mg/kg Remifemin or black tea on serum concentration of Ca, P, and ALP of ovariectomized (OVX) rats.**

| Group | Ca (U/L) | P (U/L) | ALP (U/L) |
|---|---|---|---|
| Sham | 2.89±0.14 | 2.23±0.22* | 18.33±7.51* |
| OVX | 2.71±0.18 | 2.91±0.25^{#} | 37.33±1.53^{#} |
| Ref | 2.54±0.19^{#} | 1.91±0.11* | 63.67±5.69*^{#} |
| BT | 2.37±0.03* | 1.35±0.27*^{#} | 73.67±3.79*^{#} |
| MCL100 | 2.52±0.10^{#} | 1.99±0.29* | 23.67±8.50* |
| MCL200 | 2.66±0.17 | 1.72±0.25*^{#} | 24.00±3.61* |
| MCL300 | 2.55±0.12^{#} | 1.40±0.15*^{#} | 35.00±4.00^{#} |

| | | | |
|---|---|---|---|
| Values are mean ± SD *P<0.05 vs. OVX; ^{#}P<0.05 vs. SHAM as evaluated by Duncan Test. | | | |

Inflammation markers; TNF-α and IL-6 were both increased in OVX group. Treatment with all three doses of *Morinda* suppressed the expression of TNF-α by 17.1, 17.5 and 20.8% (Figure 4a), and IL-6 by 51.3, 54.7 and 92% (Figure 4b), respectively, as compared to OVX group. Remifemin (100mg/kg) group also reduced the level of both markers by 12.2 and 44.7%. However, black tea group only reduced the level of TNF-α (33.3%) but not IL-6. The level of bone formation marker, Osteoprotogerin (OPG) was significantly reduced in OVX group by 34% compared to sham group. However, none of the treatment groups showed increase in OPG level (see Figure 4c).

Bone specific gene expressions: RANKL regulates the formation, activation and survival of osteoclasts (Boyce & Xing 2007). The level of bone resorption marker; RANKL, was increased in OVX group by 67.3% compared to sham group. All three doses of *Morinda* reduced RANKL level by 78.5, 76.3 and 67.8%, respectively. RANKL level in 100mg/kg Remifemin and black tea group were 48.2 and 79.2% lower than OVX group (see Figure 4a).

HIF-1α plays a role in promoting and accelerating osteoclast activity (Miyauchi et al. 2013), and NFkB involved in inhibiting osteoblast differentiation (Chang et al. 2009). RUNX2 and BMP-2 plays an essential role in osteoblastic differentiation and maturation (Dalle Carbonare et al. 2012; Medici et al. 2006). ER-α and COL1A1 involved in maintenance of the bone (Nakamura et al. 2007; Jin et al. 2011). As shown in Figure 4a and 4b, the expression for each marker has been normalized with OVX group (dash line). Treatment with *Morinda* downregulate the expression of bone resorbing markers; HIF-1α and NFkB and upregulate the expression of ER-α at 300mg/kg bw. At 200mg/kg, Morinda downregulated the RANKL expression and upregulated the BMP-2 expression. Treatment with Remifemin and black tea increased the expression of bone formation markers; RUNX2, ER-α and BMP-2.

Another bone formation marker; Osteocalcin (OC) level was lower in OVX group but not significant compared to sham group. Treatments with all three doses of *Morinda* significantly increased the level of OC by 24.2, 25.3 and 29.8% respectively. The level of OC in 100mg/kg Remifemin group also increased by 28.4% compared to OVX group, as shown in Figure 4b.

Figure 5 shows the bone growth effects of the herbs supplementation in ovariextomised rats (OVX) compared to control (Sham), according to the present invention on (a) femur and tibia thickness (b) Femur and tibia Bone mineral density. [G1: SHAM (control group), G2 : OVX- ( negative control) , G3 : REF (Remifemin 100 mg/kg BW), G4 : GT (2% Green tea) , G5: OSLE (*Orthosiphon spp.* ethanol extract low dose 150 mg/kg BW), G6 : OSHE *(Orthosiphon spp.* ethanol extract high dose 300 mg/kg BW), G7 : OSLA (*Orthosiphon spp.* aqueous extract low dose 150 mg/kg BW), G8 : OSHA (*Orthosiphon spp.* aqueous extract high dose 300 mg/kg BW). OPG can reduce the production of osteoclasts by inhibiting the differentiation of osteoclast precursors].

### B) Bone Calcium Density Enhancing effects From Orthosiphon spp. leaves, water and 50% ethanol standardized extract

Methods: Eleven weeks old female Sprague-Dawley rats were used and randomly divided into sham-operated group and seven ovariectomized (OVX) groups (n=7 rats/group) : OVX non-treated (OVX-) ; OVX with 2% Green tea (GT); OVX with Remifemin (100mg/kg); OVX with OS 50% ethanol extract 2 doses; 150 mg/kg (OSLE) and 300 mg/kg (OSHE); OVX with OS aqueous extract 2 doses ; 150 mg/kg (OSLA) and 300 mg/kg (OSHA); . Daily oral administration via oral gavage of all treatments began 4 weeks after the surgery and lasted for 16 weeks. Bone mass density, micro-ct scan, trabecular microarchitecture, serum biochemical markers, gene expression and H&E staining were the analysis that will be done in this study and shown to be positively affected by the Orthosiphon extracts in the ovariectomised rats.

Bone Mass Density (BMD): Figure 6 shows the effects of 16-weeks treatment with *Orthosiphon spp. (OS)* leaf aquaous (OSA) or methanol (OSM) or ethanol OSE extract [L indicate low dose (150mg/kg) and H indicates high dose of 300 mg/kg], Remifemin (REF) or Green Tea (GT) on (a) bone mass density and (b) femur diameter of ovariectomized (OVX) rats Bone mineral density (BMD) of the femur as shown and length, and (c) bone resorption marker (OPG)

Figure 7 show the effects of the herbs (Orthosiphon spp.) after 4 month supplementation on bone strength (a) maximum stress (b) Flexural (flexibility) modulus.

Proteoglycans are present in virtually all extracellular matrices of connective tissues. Proteoglycans are glycosylated proteins which are covalently attached to highly anionic glycosaminoglycans. Many forms of proteoglycans are present in virtually all extracellular matrices of connective tissues.

Figure 8a shows the Proteoglycan release and Figure 8b shows the Reactive oxygen species (ROS) in bovine cartilage explant. Reactive oxygen species (ROS) is involved in cartilage degradation. In osteoarthritis, treatments that could reduce oxidative stress are beneficial.

From Figure 8a, proteoglycan release in *Orthosiphon spp.* aqueous extract 100 µg/well (OSE 100) is the lowest compared to *Orthosiphon spp.* aqueous extract 50 µg/well (OSE 50). It shows that OSE100 might have positive effect in inhibiting proteoglycan release in osteoarthritic condition.

From Figure 8b, *Orthosiphon spp.* aqueous extract 25 µg/well (OSE 25) has the lowest ROS absorbance compared to *Orthosiphon spp.* aqueous extract 50 µg/well (OSE 50) and *Orthosiphon spp.* aqueous extract100 µg/well (OSE100).

### C) Effect of the herbs on Inhibition of Interleukin-1β-Induced Proteoglycan Degradation Cartilage Explant Culture

The Addition of IL-1β into the culture medium causing significant increases in GAG release from the joints. The amount of GAG released to the medium reflects the degree of proteoglycan degradation. Amount of GAG was determined by its reaction with 1-9-dimethyl-methylene blue method which is then measured by using the spectrophotometer instrument (or ELISA reader).

Figure 9 shows the addition of IL-1β increases the GAG release significantly. The cartilage degradation was treated with herbs; LP (Labisia spp.), CH (Citrus spp.) and GP (Gynurea spp.) in concentration of 10, 25 and 50 µg/well. Results showed LP is the best herb in treating cartilage damage and it is a better treatment compared to diclofenac whilst being at a concentration of 10 µg/well. As shown in the Figure 9, LP significantly inhibits the IL-1β effect on cartilage and is a concentration-dependent. The figure also indicates that GP have better inhibition of GAG release than CH however not a statistically significance.

IL-1β is a pro-inflammatory cytokine and a principle mediator in OA. This cytokine can induce proteoglycan depletion then causing cartilage damage or joint destruction. Current treatment of OA is able to stop inflammation and pain from OA patients however it is not a disease modifying treatment. On the other hand, herbal plant is gained interest among the researcher as it is natural and probably cheap. Malaysia is a hot and humid country, makes it a suitable place to grow herbs such as LP, GP and CH. These herbs were examined for cartilage repair potential by in-vitro study. In this study, the addition of IL-1β caused significant increase in GAG release compared with untreated control culture. The increase of GAG release is an indication of severity in cartilage damage. The inhibition of IL-1β induced GAG release is examined by the addition of treatment from herbal plants extract or commercial drug for OA (diclofenac) to the culture. Addition of LP into media completely inhibits the IL-1β induced GAG release while GP and CH have no significance in inhibition as compared with diclofenac. Thus in this in vitro study suggest that LP have better ability to protect articular cartilage from degradation.

### D) Morinda Leaves Alcoholic Extract Effects on MIA Induced Osteoarthritis Rats In Vitro And In Vivo

Figure 10a shows the inhibition of GAG release from cartilage explants by herbs. *Morinda elliptica* (ME25; 25µg/well and ME50; 50µg/well) extracts released lower GAG compared to *Morinda spp.* (MC25; 25µg/well and MC50; 50µg/well). The ME50 group is the lowest and significantly different from the than the non-treated group.

Figure 10b shows the inhibition of GAG release from cartilage explants by lower dose of *Morinda spp.* MC10; (10µg/well), which appeared more effective than MC25 or MC50. The *Morinda* treatments showed dose dependent effect as MC10 (10ug/well) had lowest GAG release compare to MC50 (50ug/well). It could be because high concentrated treatment was too concentrated and killing the chondrocytes.

Figure 10c shows the ROS expression level of cartilage explants by herbs. All treatment groups MC25 (25µg/well), MC50 (50µg/well), ME25 (25µg/well) and ME 50 (50µg/well) showed lower ROS level compared to control and non-treated groups. This shows that these treatments possessed anti-oxidative effects.

Figures 11 a to 11 g show histology H&E staining of cartilage explants from Morinda spp. and Morinda elliptica. The chondrocytes number in the treatment groups are larger than the control and non-treated groups. The nucleus size for Morinda elliptica 25µg/ml (ME25) is the biggest among the rest of treatment groups and control group and non-treated group.

### E) Clinacanthus nutans and Ficus deltoidea Extracts Chondroprotective Effects Through Inhibition of Proteoglycan and Reactive Oxygen Species Release In Cartilage Explants Induced with IL-1 b

- *Clinacanthus nutans (CN)* and *Ficus deltoidea (FD)* extracts inhibited proteoglycan release in IL-Ib induced cartilage explants

*Clinacanthus nutans* (CN) and *Ficus deltoidea* (FD) extracts inhibited proteoglycan release in IL-1b induced cartilage explants. The in vitro effect of *Clinacanthus nutans* (CN) and *Ficus deltoidea* (FD) extracts on IL-1β-induced culture were shown in bovine cartilage explants (Figure 12a). Bovine cartilage explants were cultured for 5 d with IL-IB (10ng/ml) in the presence or absence of the extracts (20, 40, and 80 ug/ml) or diclorofenac (Dic) as positive control. Results were expressed as μg of proteoglycan released into the medium per mg wet weight of the cartilage, as shown in Figure 12a. Stimulation of OA cartilage explants with IL-1β resulted in the release of matrix proteoglycans into the culture medium in quantities significantly greater than those in control cultures (94±10 ug/mg vs. 157±7 ug/mg). The release of proteoglycans from the cartilage matrix into samples treated with the plant extracts was reduced compared with the samples treated with IL-1β alone. Diclorofenac as positive control inhibited proteoglycan release significantly to 116 ug/ml. All doses (20, 40, and 80 ug/ml) of *Ficus deltoidea* extracts inhibited release of proteoglycan significantly to 120, 115, and 108 ug/ml respectively. Only the highest dose of *Clinacanthus nutans* (80 ug/ml) inhibited release of proteoglycan into media significantly to 112.6 ug/ml.
- *Clinacanthus nutans (CN)* and *Ficus deltoidea (FD)* extracts reduced release of reactive oxygen species but not nitrogen oxide in culture media of IL-1b induced cartilage explants culture

The total release of reactive oxygen species (ROS) was in accordance to the release of proteoglycan in the media (Figure 12b). Positive control, Diclorofenac exerted the most significant inhibition of total ROS release into media to 0.9 of absorbance reading. All doses (20, 40, and 80 ug/ml) of *Ficus deltoidea* extracts inhibited release of total ROS significantly to 1.1, 1.0, and 1.0 of absorbance reading respectively. Only the highest dose of *Clinacanthus nutans* (80 ug/ml) inhibited release of total ROS into media significantly to 1.1 of absorbance reading. On the other hand, all doses of *Clinacanthus nutans* (CN) and *Ficus deltoidea* (FD) extracts did not reduced release of nitrogen oxide (NO) significantly compared to non-treated IL-1b induced cartilage explant culture media, as shown in Figure 12c. It was found that only positive control, Diclorofenac significantly reduced release of NO to 0.2 of absorbance reading.
- *Clinacanthus nutans (CN)* and *Ficus deltoidea (FD)* extracts inhibited chondrocytes apoptosis and increased chondrocytes proliferation at dose 80 ug/ml

Figures 13a-13i are representative micrographs of toluidine blue stained explants (image taken at 1X magnification). The IL-1b induced cartilage explants (Figure 13b) showed to have empty lacunae compared to control (Figure 13a), which may due to chondrocytes apoptosis. Treatment of cartilage explants induced with IL-1b with Diclorofenac (Figure 13c), IL-1b with *Clinacanthus nutans* (CN) extracts at 20 ug/ml (Figure 13d), 40 ug/ml (Figure 13e) and 80 ug/ml (Figure 13f), and treatment of cartilage explants induced with IL-1b with *Ficus deltoidea* (FD) extracts at 20 ug/ml (Figure 13g), 40 ug/ml (Figure 13h), and 80 ug/ml (Figure 13i) reduced chondrocytes apoptosis, which are shown by the reduce in numbers of empty lacunae. Additionally, it was observed lacunae in cartilage explants treated with Diclorofenac (Figure 13c), 80 ug/ml of *Clinacanthus nutans* (CN) (Figure 13f) and *Ficus deltoidea* (FD) (Figure 13i) extracts contain multiple nuclei, which indicate chondrocytes proliferation.

IL-1b promotes cartilage destruction by inducing the production of proteoglycan and oxygen-derived free radicals including reactive oxygen species (ROS) and nitric oxide (NO), which initiates matrix degradation and causes chondrocyte apoptosis. The significant increase in proteoglycan released from the cartilage explants in response to the IL-1b shows that IL-1b effectively mimicked the cytokine-induced glycosaminoglycan loss observed in OA. Modifying the IL-1b-induced proteoglycan losses is thought to be protective for the cartilage and could serve as a target for the development of therapeutics for OA.

### INDUSTRIAL APPLICABILITY

As described hereinbefore, the extract of Morinda, Orthosiphon, Clinicanthus, Ficus, Labisia and Gynurea spp. of the present invention has effect of enhancing bone growth, prevent bone resorption disorders and for joint health (anti-arthritis). Thus, the extract and mixture extracts of the present invention, owing to its synergy effect, can be effectively used for the prevention and treatment osteopenia, arthritis, bone and joint ailments or bone fracture as medicinal agent and health food.

### Numbered embodiments

1. A composition for preventing or treating osteopenia, arthritis, bone and joint ailments or bone fracture, including enhancing bone growth, preventing bone resorption disorders and joint health (anti-arthritis), which contains Morinda, Orthosiphon, Clinicanthus, Ficus, Labisia and Gynurea spp. extract.
2. A composition according to numbered embodiment 1, wherein the extract is extracted with a solvent selected from the group consisiting of water, alcohol, an organic solvent, and mixtures thereof.
3. A composition according to numbered embodiment 1, wherein the composition further comprising polyphenols, catechins, *scopoletin, sinensetin,* flavonoids and antioxidants.
4. A health functional food preventing or treating osteopenia, arthritis, bone and joint ailments or bone fracture, including enhancing bone growth, preventing bone resorption disorders and joint health (anti-arthritis), which contains Morinda, Orthosiphon, Clinicanthus, Ficus, Labisia and Gynurea spp. extract.
5. A health functional food according to numbered embodiment 4, wherein the extract is extracted with a solvent selected from the group consisiting of water, alcohol, an organic solvent, and mixtures thereof.
6. A health functional food according to numbered embodiment 4, further comprising polyphenols, catechins, *scopoletin, sinensetin,* flavonoids and antioxidants.
7. A method for preparing an extract according to numbered embodiment 1, wherein the method comprising the steps of:
   (i) finely cutting a whole plant of Morinda, Orthosiphon, Clinicanthus, Ficus, Labisia and Gynurea spp.;
   (ii) extracting the cut plant with a solvent selected from the group consisting of water, an organic solvent , and mixtures thereof, thereby obtaining an extract; and
   (iii) filtering the extract and concentrating the filtered extract under reduced pressure, thereby obtainng a Morinda, Orthosiphon, Clinicanthus, Ficus, Labisia and Gynurea spp. extract.
8. A method according to numbered embodiment 7, further comprises the step of:
   (iv) purifying the Morinda, Orthosiphon, Clinicanthus, Ficus, Labisia and Gynurea *extract* obtained in step (iii), using activated carbon and concentrating the purified extract under reduced pressure.
9. A Morinda, Orthosiphon, Clinicanthus, Ficus, Labisia and Gynurea spp. extract, which is prepared by a method of any one of numbered embodiments 7 to 8 and has bone growth enhancing bone effects, bone resorption disorder preventive effects and joint health (anti-arthritis) promoting effects.
10. Use of a composition comprising Morinda, Orthosiphon, Clinicanthus, Ficus, Labisia and Gynurea spp. extract as an active ingedient in the manufacture of a medicament for preventing or treating osteopenia, arthritis, bone and joint ailments or bone fracture, including enhancing bone growth, preventing bone resorption disorders and joint health (anti-arthritis).
11. The use according to numbered embodiment 10, wherein the composition further comprising at least one pharmaceutically acceptable carrier, excipient or diluent.
12. The use according to numbered embodiment 10, wherein the extract is extracted with a solvent selected from the group consisting of water, an organic solvent, and mixtures thereof.

## Claims

1. A composition for use in preventing or treating osteopenia, arthritis, bone and joint ailments or bone fracture, including enhancing bone growth, preventing bone resorption disorders and joint health (anti-arthritis), which contains Morinda, Orthosiphon, Clinicanthus, Ficus, Labisia and Gynurea spp. extract.

2. A composition for use according to claim 1, wherein the extract is extracted with a solvent selected from the group consisting of water, alcohol, an organic solvent, and mixtures thereof.

3. A composition for use according to claims 1 or 2, wherein the composition further comprising polyphenols, catechins, *scopoletin, sinensetin,* flavonoids and antioxidants.

4. A composition for use according to claims 1 to 3, wherein the composition is a health functional food.

5. A composition for use according to any preceding claim, wherein the composition further comprises at least one pharmaceutically acceptable carrier, excipient or diluent.

6. A method for preparing an extract according to any preceding claim, wherein the method comprises the steps of:
(i) finely cutting a whole plant of Morinda, Orthosiphon, Clinicanthus, Ficus, Labisia and Gynurea spp.;
(ii) extracting the cut plant with a solvent selected from the group consisting of water, an organic solvent, and mixtures thereof, thereby obtaining an extract; and
(iii) filtering the extract and concentrating the filtered extract under reduced pressure, thereby obtaining a Morinda, Orthosiphon, Clinicanthus, Ficus, Labisia and Gynurea spp. extract.

7. A method according to claim 6, further comprising the step of:
(iv) purifying the Morinda, Orthosiphon, Clinicanthus, Ficus, Labisia and Gynurea *extract* obtained in step (iii), using activated carbon and concentrating the purified extract under reduced pressure.

8. A Morinda, Orthosiphon, Clinicanthus, Ficus, Labisia and Gynurea spp. extract, which is prepared by a method of any one of claims 6 to 7 and has bone growth enhancing bone effects, bone resorption disorder preventive effects and joint health (anti-arthritis) promoting effects.

9. Use of a composition comprising Morinda, Orthosiphon, Clinicanthus, Ficus, Labisia and Gynurea spp. extract as an active ingredient in the manufacture of a medicament for preventing or treating osteopenia, arthritis, bone and joint ailments or bone fracture, including enhancing bone growth, preventing bone resorption disorders and joint health (anti-arthritis).

10. The use according to claim 9, wherein the composition further comprising at least one pharmaceutically acceptable carrier, excipient or diluent.

11. The use according to claims 9 or 10, wherein the extract is extracted with a solvent selected from the group consisting of water, an organic solvent, and mixtures thereof.
